# EUROPEAN PATENT SPECIFICATION

(11) **EP 2 474 625 B1**
(45) Date of publication and mention of the grant of the patent: **02.11.2016**
(21) Application number: 11150234.0
(22) Date of filing: 05.01.2011
(51) Int. Cl.: C12Q 1/68

(54) **Method for determining the p53 status of a tumour**
Verfahren zur Bestimmung des p53-Status in Tumoren
Procédé de détermination du statut du gène p53 d'une tumeur

(43) Date of publication of application: 11.07.2012
(73) Proprietor: Kandioler, Daniela, 3032 Eichgraben (AT)
(72) Inventor: Kandioler, Daniela, 3032 Eichgraben (AT)
(74) Representative: Sonn & Partner Patentanwälte

(56) References cited:
- WO-A1-98/59072
- WO-A1-2012/093152
- WO-A2-00/70085
- US-A- 6 071 726
- BÄCKVALL HELENA ET AL: "Mutation spectra of epidermal p53 clones adjacent to basal cell carcinoma and squamous cell carcinoma.", EXPERIMENTAL DERMATOLOGY OCT 2004 LNKD- PUBMED:15447725, vol. 13, no. 10, October 2004 (2004-10), pages 643-650, XP002638893, ISSN: 0906-6705
- KANDIOLER-ECKERSBERGER D ET AL: "TP53 mutation and p53 overexpression for prediction of response to neoadjuvant treatment in breast cancer patients", CLINICAL CANCER RESEARCH, THE AMERICAN ASSOCIATION FOR CANCER RESEARCH, US, vol. 6, no. 1, 1 January 2000 (2000-01-01) , pages 50-56, XP2633764, ISSN: 1078-0432
- LEHMAN T A ET AL: "P53 MUTATIONS RAS MUTATIONS AND P53-HEAT SHOCK 70 PROTEIN COMPLEXES IN HUMAN LUNG CARCINOMA CELL LINES", CANCER RESEARCH, vol. 51, no. 15, 1991, pages 4090-4096, XP002638894, ISSN: 0008-5472
- AGELL LAIA ET AL: "KLF6 and TP53 mutations are a rare event in prostate cancer: distinguishing between Taq polymerase artifacts and true mutations", MODERN PATHOLOGY, vol. 21, no. 12, December 2008 (2008-12), pages 1470-1478, XP002638895, ISSN: 0893-3952
- SONG JAEHWI ET AL: "Genetic and epigenetic alterations of the KLF6 gene in hepatocellular carcinoma", JOURNAL OF GASTROENTEROLOGY AND HEPATOLOGY, vol. 21, no. 8, August 2006 (2006-08), pages 1286-1289, XP002638896, ISSN: 0815-9319
- KANDIOLER DANIELA ET AL: "Growing clinical evidence for the interaction of the p53 genotype and response to induction chemotherapy in advanced non-small cell lung cancer.", THE JOURNAL OF THORACIC AND CARDIOVASCULAR SURGERY MAY 2008 LNKD- PUBMED:18455581, vol. 135, no. 5, May 2008 (2008-05), pages 1036-1041, XP0022646773, ISSN: 1097-685X
- ROUX K H: "Optimization and troubleshooting in PCR.", PCR METHODS AND APPLICATIONS APR 1995, vol. 4, no. 5, April 1995 (1995-04), pages S185-S194, ISSN: 1054-9803
- Gao Ling ET AL: "PATCHED and p53 gene alterations in sporadic and hereditary basal cell cancer", Oncogene, vol. 20, no. 53, 22 November 2001 (2001-11-22), pages 7770-7778, XP055204290, ISSN: 0950-9232, DOI: 10.1038/sj.onc.1204946

## Description

The invention relates to the field of tumour diagnosis, especially with respect to use such diagnosis for appropriate therapy decisions.

Tumour diseases (or cancers (cancer diseases), i.e. malignant neoplasms) is a class of diseases in which a group of cells displays uncontrolled growth (division beyond the normal limits), invasion (intrusion on and destruction of adjacent tissues), and sometimes metastasis (spread to other locations in the body via lymph or blood). These three malignant properties of cancers differentiate them from benign tumours, which are self-limited, and do not invade or metastasise. Most cancers form a (solid) tumour mass but some, like leukemia, do not.

Tumour diseases affect people at all ages with the risk for most types increasing with age. Such diseases cause a rising number of deaths; in most countries between 10 and 30 % of all human deaths.

Tumour diseases are caused by abnormalities in the genetic material of the transformed cells. These abnormalities may be due to the effects of carcinogens, such as tobacco smoke, radiation, chemicals, or infectious agents. Other tumour disease-promoting genetic abnormalities may randomly occur through errors in DNA replication, or are inherited, and thus present in all cells from birth. The heritability of tumour diseases is usually affected by complex interactions between carcinogens and the host's genome.

Therapy of a tumour disease (also referred to as: cancer management options) is currently performed in many ways, the most important being chemotherapy, radiation therapy, surgery, immunotherapy and monoclonal antibody therapy. The choice of therapy depends upon the location and stage of the tumour and the grade of the disease, as well as the general state of a person's health. Experimental cancer treatments are also under development. It is also common to combine more than one therapy for the treatment of a tumour patient.

Complete removal of the tumour without damage to the rest of the body is the goal of treatment. Sometimes this can be accomplished by surgery, but the propensity of the tumour disease to invade adjacent tissue or to spread to distant sites by microscopic metastasis often limits its effectiveness. Surgery often required the removal of a wide surgical margin or a free margin. The width of the free margin depends on the type of the cancer, the organ affected, and the method of removal. The effectiveness of chemotherapy is often limited by toxicity to other tissues in the body. Radiation can also cause damage to normal tissue.

When describing effects of treatment regimens it is important to determine the direction of between-treatment difference among patient's subsets. Effects from qualitative and quantitative interaction have to be distinguished (Gail et al., Biometrics 41 (1985), 361-372).

A quantitative interaction occurs if the treatment effect varies in magnitude but not in direction across all patient subgroups. It is frequently referred to as non-crossover interaction and leads to a therapy effect in responders and no effect in non-responders. So in some patients the therapy may not help, but does not do harm either.

In case of a qualitative interaction (crossover interaction) the between-treatment difference changes direction among patient subsets. This means that the application of a certain therapy improves outcome of some patients (responder) and has an inverse effect on other patients. A qualitative interaction is the strongest interaction known between treatment and patient outcome and creates great differences between groups (Gail et al., 1985).

A qualitative interaction profoundly influences patient outcome and trial outcome if not realised.

The concept of qualitative interaction is statistically known (Gail et al., 1985). A qualitative interaction between a marker and treatment outcome has not yet been described in cancer therapy.

The proof of a qualitative interaction generates demand for marker testing due to ethical and safety considerations.

It is part of the present invention to improve effectiveness of treatment of tumour diseases. A specific aspect is the prevention of worsening the status of a cancer patient by choosing the wrong treatment strategy, i.e. to prevent a negative effect of a tumour therapy which is (although being effective in some patients) harming the patient being treated with a treatment not being appropriate for the tumour.

Therefore, the present invention includes a method for determining the p53 status of a tumour patient as defined in the claims.

The present invention is based on the identification of a qualitative interaction between the marker p53 and response to the treatment of the tumour disease.

The p53 tumour suppressor is a 393-aa transcription factor. In response to various types of genotoxic stresses, p53 transactivates a number of genes by binding to specific DNA sequences, thereby arresting cell cycle, repairing damaged DNA, or inducing apoptosis as the cell fates. The structure of the p53 core DNA-binding domain (residues 94-312) that binds directly to the DNA sequence has been resolved by x-ray crystallography, and both x-ray crystallography and NMR analysis have been used to deduce the structure of the tetramerisation domain (residues 323-356), which is needed for optimum function. The structure of the p53 Protein is composed of 6 functional domains. The amino-terminal residues one to 42 and 43 to 63 contain two transactivation domains. The first one can be bound by MDM2, a negative regulator of p53 and the second one can bind to p53-responsive elements in promoters of different p53-regulated genes to activate their transcription. The proline-rich domain spanning residues 61-94 is involved in apoptosis and protein-protein interactions. The largest domain including residues 102-292 functions in binding p53-responsive sequences associated with genes regulated by p53. The p53 protein functions as tetramer. Tetramerization is accomplished by residues 324-355. The carboxy-terminal domain from residue 363 to 393 regulates the stability and DNA binding activity of the p53 protein (reviewed by. Belyi et al., Cold Soring Harbor Perspectives in Biology 2010;2:a001198). The p53 activity is regulated by posttranslational mechanisms such as phosphorylation, methylation, acetylation, and prolylisomerisation, or by protein-protein interaction, thereby it becomes stabilised and can conduct its respective physiological function (reviewed by Olsson et al., Cell death and Differentiation 14 (2007), 1561-1575).

Somatic TP53 mutations are the most common (about 50%) genetic alteration in human cancer, and a large number of TP53 mutations have been assembled in TP53 mutation databases. The latest International Agency for Research on Cancer (IARC R14 from 2009) TP53 mutation database contains 26597 somatic mutations and 535 germ-line mutations. Among these, 89.8% of p53 mutations are clustered in the core DNA-binding domain and over 70% of the mutations are missense mutations. So far, 4235 distinct mutations including 1586 amino acid substitutions caused by missense mutations have been documented. Compared to all mutations published, there is little increase in the number of newly described mutations since the year 2002 (10% per year) and even less increase in the number of newly described amino acid substitutions (2.85% per year) (IARC p53 mutation database release R14 from November 2009; Petitjean et al. Hum Mutat. 28 (2007), 622-629). Therefore it seems that most tumour associated missense mutations have been already identified and unreported missense mutations might be non-pathogenic for tumour development. Furthermore 2314 p53 mutants representing all possible amino acid substitutions caused by a point mutation throughout the coding sequence have been evaluated using a functional assay (Kato et al., PNAS 100 (2003), 8424-8429).

Due to its important role in tumour biology, p53 has been in the focus of tumour diagnosis, and especially as a potential predictive marker for therapy response.

The initial observation that p53 accumulation might serve as a surrogate biomarker for TP53 mutation has been the cornerstone for vast translational efforts aimed at validating its clinical use for the diagnosis, prognosis, and treatment of cancer. Early on, it was realised that accurate evaluation of p53 status and function could not be achieved through protein-expression analysis only. As the understanding of the p53 pathway has evolved and more sophisticated methods for assessment of p53 functional integrity have become available, the clinical and molecular epidemiological implications of p53 abnormalities in cancers are being revealed. They include diagnostic testing for germline and somatic p53 mutations, and the assessment of selected p53 mutations as biomarkers of carcinogen exposure and cancer risk and prognosis. The strengths and limitations of the most frequently used techniques for determination of p53 status in tumours, as well as the most remarkable latest findings relating to its clinical and epidemiological value are described most recently by Robles et al. (CSH Perspect. Biol. 2 (2010), a001016). The most important methodologies for assessment of p53 status in clinical and epidemiological studies are DNA sequencing, immunohistochemistry (IHC), TP53 mutational load assay, mass spectrometry, microarray analysis and functional analysis (reviewed by Robles et al., 2010).

DNA sequencing is an established method for identification of TP53 mutations and often the method of choice for such purpose. Gel-based mutation screening assays, such SSCP or PCR-RFLP, are routinely used before sequencing. In this technique, TP53 is amplified and resulting PCR fragments are subjected to enzymatic restriction using an enzyme for which a site is predicted to be created or destroyed by the presence of mutation. The resulting gel profile after enzymatic restriction or due to denaturising conditions is used as an indicator for the presence of mutation, and sequencing of that area is undertaken using direct sequencing methods. Most TP53 mutations identified in tumours are circumscribed to the area encompassing exons 5-8 and therefore many translational studies have limited their mutational analysis to this portion of the gene. This has caused bias in the TP53 mutation literature because mutations outside exons 5-8 may have been missed. Additionally mutations may have been missed due to the limited sensitivity of the screening techniques. The problem is likely to be solved with more sophisticated targeted high-throughput DNA sequencing strategies that may in the future be used to gather nucleotide-level information about TP53 and other critical genes in clinical samples. Still, the presence of mutation does not unequivocally indicate that p53 is fully inactive, nor does the absence of it indicate that p53 is functionally proficient. Thus, assessing functional activity of p53 mutants was regarded as essential for an accurate indication of clinical relevance (Robles et al., 2010).

In the present case, however, the assessment of p53 status of a given tumour is of central importance for the decision concerning the appropriate treatment strategy for the patient. Defining a p53 status of a patient's tumour, i.e. assessing whether the tumour has a mutation in the p53 gene or not is the most critical issue for the present invention in order to deliver the appropriate anticancer drugs to a specific patient.

Most of the currently applied anticancer therapies use one of two different pathways to attack cancer cells: They either act via apoptosis or interfere with the cell cycle. p53 is crucially involved in both pathways:
Pathway 1: cancer therapy induces DNA damage and subsequent apoptosis: DNA damage is a strong trigger for p53 activation. Activated p53 transactivates apoptosis genes which lead to cell death. This mechanism has been suggested e.g. for drugs acting as antimetabolites, antibiotics or alkylating agents (not for drugs acting in the M phase).
Pathway 2: cancer therapy interferes with (different phases of) the cell cycle: In case of p53 mutation, cells cannot be controlled (arrested) in G1 phase of the cell cycle. Cells are cycling unbreakable. Therefore more cells are in S or M phase which makes them sensitive for cell cycle interfering drugs (synchronization effect).

In case of p53 the marker identifies a patient subset (s a) which will not be treated successfully but will be harmed by a certain type of therapy. At the same time the p53 status of the tumour determines potentially effective therapies (other pathway) for this subset of patients. It follows that normal p53 enhances the activity of apoptosis inducing cancer therapy but impairs activity of cell cycle interfering agents; it also follows that mutant p53 enhances activity of cell cycle interfering cancer therapy but impairs activity of apoptosis inducing agents.

**Table 1: Association between p53 status and response to cancer therapy.**

| | Pathway 1 Apoptosis | Pathway 2 Cell cycle |
|---|---|---|
| p53 normal | enhance | impair |
| p53 mutant | impair | enhance |

The p53 status of a tumour determines which type of therapy will be successful but also which therapy will harm the patient. The qualitative interaction describes the variation in direction of the therapy effect (one therapy is superior for some subsets and the alternative treatment is superior for the other subsets). Additionally there is an inverse magnitude of effect (= wrong treatment harms patients which can be seen in survival curves showing worse outcome for patients receiving a "non p53 adapted therapy" when compared to those receiving no treatment (i.e. in case of non p53 adapted therapy the treatment does not help but harms).

With the present invention it should be safeguarded that the tumour patient receives the appropriate treatment and - even more important - is protected from suffering the negative impact of the wrong treatment.

Despite the considerable knowledge about assessment of p53 status of a given tumour patient, there is still the need for a reliable p53 diagnosis in the field. One of the objects of the present invention is the provision of a reliable method for assessment of the p53 status of a given tumour patient.

WO 98/59072 A2 discloses a kit for multiplex PCR of i.e. p53. Bäckvall et al. (Exp. Dermatol. 13 (2004), 643-650) use multiplex PCR amplification as pre-amplification to enrich p53 DNA fragments, followed by PCR amplification of each exon in individual reactions prior to sequences. In Kandioler-Eckersberger et al. (Clin. Can. Res. 6 (2000), 50-56) only PCR tests wherein mutations were detected were repeated. Lehmann et al. (Cancer Res. 51 (1991), 4090-4096) disclose PCR tests involving some p53 intron regions. Agell et al. (Mod. Pathol. 21 (2008), 1470-1478) disclose a p53 PCR test only involving exons 4 to 9. Song et al. (J. Gastroent. Hepatol. 21 (2006), 1286-1289) describe a PCR test involving exon 2 of KLF6 gene. Kandioler et al. (J. Thor. Cardiovasc. Surg. 135 (2008), 1036-1041) disclose a routine p53 PCR test; further routine p53 tests are disclosed e.g. in US 6 071 726 A and WO 00/70085 A2.

Therefore, the invention provides a method for determining the p53 status of a tumour patient as defined in the claims.

The method according to the present invention allows a reliable answer to the question whether the tumour cells or cell-free tumour DNA tested carry a mutation in their p53 gene or not. This is specifically advantageous on the decision for the optimal tumour treatment, especially in view of the qualitative interaction with respect to p53 (see below). With the method according to the present invention, the p53 gene is sufficiently covered so that no false negative or false positive result (which would cause wrong decisions for treatment of the tumour patient) is practically possible. The method is adapted to the needs of practical diagnosis and is suitable for large number testing performed in clinical trials and also in everyday clinical practice.

The present method is based on the reliable determination of the genetic p53 status of a given tumour cell of a tumour patient. This requires the provision of a sample of tumour cells of this patient. Preferably, for defining the p53 status of a patient's tumour a sample of body fluid or a tissue sample of the patient is used which is a blood sample or a tumour biopsy sample (containing histologically verified tumour cells). The present invention provides a reliable determination of the genetic p53 status of a given tumour cell of a tumour patient which requires the provision of a sample of tumour cells of this patient and subjecting this tumour sample to the method according to the present invention, i.e. finding whether a p53 mutation is present in the tumour cell DNA or not. Such a sample can be a tissue specimen, e.g. a tumour biopsy or a suspension of tumour cells harvested by any method, or a sample of a body fluid from such a patient, such as blood (or a blood derived sample, such as serum or plasma), cerebrospinal fluid, lymph, ascitic fluid, or any other body-derived liquid containing tumour cells. The "tumour status" of such cells has to be verified first either by histological or biochemical (immunological) or genetic verification or other means of verification.

The term "quality controlled" has to be understood in that the performance of the PCR is controlled during the reaction. This means that at least one negative control is provided and that the PCR products are analysed (preferably by electrophoretic methods, especially gel electrophoresis). The negative control is preferably a PCR set up with water instead of the DNA (of the sample); of course, also other negative controls can be foreseen, e.g. DNA which should not be polymerised in the PCR can be used as negative control. The negative control serves as a quality control for the exactness of the PCR as well as whether contaminations are present in the stock solutions for the chemicals or in the instruments used; the analysis of the PCR products (especially with respect to their size e.g. by gel electrophoresis) also serves for identifying contaminations or artefacts in the PCR which can interfere with the sequencing step.

The term "quality control" according to the present invention (preferably) also includes that the content of tumour cells histologically verified, the coverage of the p53 gene (amplification of exon 2-11 + intron regions), the triplicate PCR and sequencing; the forward and reverse sequencing, the additional visual inspection, especially by experienced personnel, etc..

The present invention is applicable for all types of tumour diseases, i.e. for all cancer patients.

Accordingly, preferred tumour diseases for which the p53 status is determined according to the present invention are solid tumours, especially colorectal cancer, esophagus cancer, gallbladder cancer, lung cancer, breast cancer, oral cancer, ovarian cancer, pancreas cancer, rectal cancer, gastrointestinal cancer, stomach cancer, liver cancer, kidney cancer, head and neck cancer, cancer of the nervous system, retinal cancer, non-small cell lung cancer, brain cancer, soft tissue cancer, lymphnode cancer, cancer of the endocrine glands, bone cancer, cervix cancer, prostate cancer or skin cancer; or a haematological tumour, preferably acquired aplastic anaemia, myelodysplastic syndrome, acute myeloid leukaemia, acute lymphatic leukaemia, Hodgkin lymphoma, non-Hodgkin lymphoma or multiple myeloma.

The multiplex format allows a cost-effective performance of the method without being too time or workload consuming. Generally, a multiplex-PCR consists of multiple primer sets within a single PCR mixture to produce amplicons of varying sizes that are specific to different DNA sequences within the p53 gene. By targeting multiple genes at once, additional information can be gained from a single test run that otherwise would require several times the reagents and more time to perform.

However, the problem of applying multiplex PCR set-ups in clinical practice is often a lack of reliability and a lack of standardisation ability. Therefore, the method according to the present invention uses a quality-controlled multiplex PCR format which includes a triplicate performance of each PCR test. "Triplicate" according to the present invention means that routinely at least three PCR tests are performed for each set-up, i.e. "triplicate" includes not only "three times" but also "four times", "five times", "ten times" or even more. A person skilled in the art understands that the triplicate multiplex set up according to the present invention sets a new quality standard for p53 testing and that performance and reliability can still be further enhanced by even more parallel set ups; however, the number of set ups has to be weighed with cost and performance considerations. For the method according to the present invention, triplicate performance has proven to be necessary and sufficient for the reliability needed; triplicate testing has to deliver three identical results. If this is not the case, a 10 time testing will allow either a decision or uncover the reason why the test is inconsistent in this certain probe.

Duplicate testing has turned out to be not reliable enough for a standard medical testing method used in clinical practise. On the other hand, e.g. quadruplicate or quintuplicate testing can be even more reliable but such strategy adds costs and effort.

For prevention of false negative results DNA has to be taken separately for each of the triplicate PCR.

For identification of false positive results, negative controls can be foreseen, e.g. by a PCR set up with the same reagents as the samples, but without DNA. If a negative control shows a PCR product, the whole set up must be repeated with new aliquots of reagents and after a (UV-) sterilisation of the work bench.

Annealing temperatures for each of the primer sets must be optimised to work correctly within a single reaction, and amplicon sizes, i.e., their base pair length, should be different enough to form distinct bands when visualised by gel electrophoresis. The amplified nucleic acid must also be suitable for sequencing, e.g. by automated DNA sequencing machines and also other sequencing methods.

The coverage of the p53 gene according to the present invention was carefully chosen to prevent the miss of relevant mutations. Therefore, the method according to the present invention covers at least exon 2 to exon 11 of the p53 gene of the EMBL sequence U94788 (Seq.ID No. 1) thereby also including introns 2 to 10, preferably at least 30 bp adjacent to the respective exon, in order to check all portions of the gene where mutations can eventually be present and relevant for p53 function. Prior art methods have often only observed more specific parts of the p53 gene. Unknown or infrequent mutations in other regions have been missed by such practice. This is excluded by the method according to the present invention. The region including exon 2 to exon 11 (i.e. the region from bp 11619 to bp 18741 (covering all amplicons used in the most preferred embodiment), especially from bp 11689 to bp 18680) of the p53 gene is specifically suitable for the method according to the present invention allowing a robust and comprehensive testing of all relevant portions of the p53 gene. A preferred embodiment of the present invention is characterised in that primers are used for amplification of the p53 gene which also include regions (in the resulting amplified molecule) which are at least 10, preferably at least 20, especially at least 30 bp, adjacent to the respective exon. It is, of course also possible to include regions which are at least 50, at least 80, or at least 100 bp (or even more), adjacent to the respective exon. Such primers have significant advantages to primers (such as the IARC primers) which do not allow characterisation of all parts of the exon after forward and reverse sequencing. Moreover, with the preferred primers according to the present invention, intron regions are included in which splice site mutations can occur.

The set of primers used in the present method includes all primers according to SEQ.ID.Nos. 2 to 24.

With the multiplex PCR according to the present invention multiplex PCR amplification products are generated. The sequence of these amplification products ("amplicons") is determined by DNA sequencing. The most convenient and appropriate method for sequencing is automated DNA sequencing. Sequencing according to the present invention is performed by using forward and reverse primers for sequencing for each of the three (or more) multiplex-PCR products. This is also a quality feature and prevents false results, because some mutations can be overlooked if sequencing was performed in the forward or in the reverse only. The result of the sequencing step is the determination of the exact sequence of the p53 gene in the region of said tumour cells which has been amplified by the multiplex PCR.

The comparison of the generated sequence with a native p53 gene sequence (which definitely does not have mutations in the p53 gene) finally allows to come to the result of the present test, namely the identification of one or more specific mutations in the p53 gene or the verification that the cancer cell tested does not have a mutation in the p53 gene. This comparison can be done automatically by various computer programs; however it is an additional and preferred quality control step to inspect the sequences visually, e.g. by experienced sequencing experts, in order to interpret suboptimal or inconclusive data and/or to make the decision for resequencing. Usually, after finishing the sequence run the raw data (e.g. the fluorescence signals) can be stored, analysed and transferred in a sequence format. Depending on the program used, the raw data (e.g. SeqScape) or the analysed sequence (e.g. Autoassembler, SeqScape) are used for the comparison. However, the method according to the present invention is not dependent on a specific sequencing platform and can be applied in any sequencing method (ABI, Beckmann, etc.), as long as a comparison of a multitude of (at least more than one) sequence runs can be performed on different samples and compared with each other.

The p53 status of a tumour patient will be determined as mutated if at least one mutation was detected in the nucleic acids of said tumour cells by the method according to the present invention. If no mutation was detected, the p53 status of this patient will be determined to be native. Overall therapy depends on the primary tumour, the primary tumour is therefore the basis for the assay according to the present invention. If a tumour shows synchronic metastasis, p53 status of the metastases is unchanged. If, nevertheless, different mutation status should occur in a patient this could be due to two different primary tumours. In such exceptional cases, the two possible optimal therapy regimes have to be fine-tuned to each other (e.g. local irradiation for the non-mutated tumour and pathway 2 therapy for mutated tumours; or sequential administration of chemotherapies).

According to a preferred embodiment, the multiplex PCR in the method according to the present invention is performed with primers having a melting temperature of 58°C to 72°C, preferably of 60°C to 70°C, especially of 65°C to 68°C. This temperature/primer combination is especially suitable for standard testing in clinical practice. Optimum melting temperatures can be determined for a given primer set by a person skilled in the art, mainly based on the primary sequence to be analysed and on the salt concentration of the buffers.

Multiplexing the PCR allows a time and effort saving performance of the method according to the present invention. However, care must be taken that the PCR is not "overloaded" with primers and sample DNA, because this could lead to false negative results (if a given sub-reaction did not properly work) or amplification artefacts (which could produce a high background signal and interference with sequence analysis). Care must be taken to adjust appropriate number of different amplifications in one reaction, the lengths of the amplicons, the number of PCR cycles, etc. Multiplex PCR is performed with at least two different primer pairs (= four primers); such multiplex PCR resulting in at least two independent PCR products. The multiplex PCR according to the present invention is preferably performed with a total (for a given test of a patient's cells) of at least 8 or of at least 10, preferably at least 15, especially at least 20, primer pairs covering different regions of the p53 gene. These primer pairs are then provided in combinations of two primer pairs or more in suitable multiplex set-ups. In order to make the multiplex PCR according to the present invention efficient with respect to time and effort, the totality of the primers in the multiplex PCR is performed with 5 or less independent PCRs, more preferred with 4 or less independent PCRs, especially with 3 or less independent PCRs. Having a number of at least 10, especially at least 20 primer pairs, provision of three independent PCRs has shown to be the most preferred embodiment; only two or even only one PCR reaction for all the primer pairs has drawback with respect to complexity, especially in obtaining the results and compatibility with the sequencing step thereafter.

A primer set has been developed for the present method which provides specifically suitable reliability and performance for determination of the p53 status of a tumour patient. This primer set has been designed for the clinical testing of the present invention and therefore fully serves the needs of the present invention. Therefore, according to a preferred embodiment of the present invention, these primers are used for carrying out the present invention. The method according to the present invention is therefore characterised in that the primer pairs according to SEQ.ID.Nos. 2 (use of primer 3 could result in a reduced distinguishability of the amplicon) and 4 to 22 is/are used in said triplicate multiplex PCR and/or said sequence determination. With the exception of the primers for exon 4 (SEQ.ID.Nos. 7 and 8), these PCR primers are also used in the most preferred embodiment for sequencing (for exon 4, the sequencing primers SEQ.ID.Nos. 23 and 24 are used).

A specifically preferred embodiment of the invention is characterised in that the primer pairs according to SEQ.ID.Nos. 2 to 24 are used in said triplicate multiplex PCR and/or said sequence determination (again, with the peculiarities concerning exon 4). It is also clear to a person skilled in the art that the primers herein can be slightly amended (e.g. shifting some (e.g. 1, 2, 3, 4, or 5) base pairs 5' or 3' along the p53 sequence) usually without much difference, nevertheless, the primers disclosed herein represent a specifically preferred embodiment.

Preferably, the p53 status determination according to the present invention is performed by running in parallel at least a positive and/or a negative control. Preferred positive controls are a tumour cell or a cell-free DNA with a p53 gene in native form and/or a tumour cell or a cell-free DNA with a mutated p53 gene. Preferably, the nature and details of the p53 mutation is known; also DNA with a p53 gene with more than one mutation can be applied as a positive control. Such positive controls are useful as markers for the appropriate working of the amplifications and/or the detectability of wild type or mutant p53 gene. Preferred negative controls run in parallel to the determination of the p53 status of the tumour patient are DNA free of sequences that are amplified during the triplicate multiplex PCR and/or a DNA free solution. The "DNA free of sequences that are amplified during the triplicate multiplex PCR" is a DNA which, under appropriate working of the PCR reaction does not result in any amplification product with the specific primers applied. Creation of an amplification signal in such negative control implies then either contamination by another DNA or unsuitable PCR conditions (too low stringency; too low polymerase specificity, etc.). It is clear that positive and negative control PCRs have to be carried out identically (stringency, polymerase specificity, etc.) to the sample PCRs. As already stated above, it is preferred to use the same primers for a given triplicate multiplex PCR and for the determination of the sequence of said triplicate multiplex PCR amplification products, i.e. the sequencing primers for a given amplicon are the same as for the PCR. This applies for most of the primers/amplicons (except for exon 4, where the amplicon spans a repetitive sequence which has to be excluded in sequence analysis). Accordingly, at least one, preferably at least three, especially at least five primer pairs for the PCR are also used for the sequencing.

Another aspect of the present invention relates to a kit for performing the method according to the present invention. The kit according to the present invention comprises:
- a PCR primer set with all primer pairs (forward/reverse) of SEQ.ID.Nos. 2 to 24;
- optionally, PCR reagents, including a DNA polymerase, buffer(s), and dNTPs; and
- a sequencing primer set.

This kit can be packaged and provided in a "ready to use" format so that it is applicable in any diagnosis laboratory to determine the p53 status of a tumour patient. PCR reagents, including a DNA polymerase, buffer(s), and dNTPs, can be provided in the kit; however, it is also established practice that such reagents are supplied separately (e.g. ingenetix MSI Panel PCR Kit), so that the PCR kits are commercialised with the primers only. Of course, for performing the method according to the present invention, these reagents have to be present.

In a preferred embodiment, the kit according to the present invention further comprises control reagents, preferably positive control reagents, especially a tumour cell or a cell-free DNA with a p53 gene in native form and/or a tumour cell or a cell-free DNA with a mutated p53 gene, or negative control reagents, DNA free of sequences that are amplified during the triplicate multiplex PCR and/or a DNA free solution.

Often, the PCR reagents and primers as well as the polymerase are optimised with respect to a given thermocycler. It is therefore practical, if the kit of the present invention also comprises a thermocycler ready to be used with the other components of the kit. Preferably, the other components of the kit, especially the buffers, PCR primers and the polymerase have been optimised for the given thermocycler.

Preferred kits of the present invention already contain the primers in prepared multiplex mixtures so that the primers do not have to be added separately to the PCR mix but are already provided in the appropriate multiplex mixture (i.e. in the optimised concentrations).

As stated above, no marker for a qualitative interaction has yet been described in the prior art in cancer treatment. In the course of the present invention, however, p53 was identified as such marker for qualitative interaction. Accordingly, a new method for diagnosing a tumour patient was established which is essentially based on the p53 status of a given cancer patient (see above).

This method, of course, heavily depends on a reliable method for determining the p53 status of a given patient to positively use the qualitative interaction between the marker p53 and response to the treatment of the tumour disease.

In the prior art, a number of p53 testing techniques have been disclosed. However, the present invention relies on the multiplex PCR format and gene sequencing which surprisingly turned out to provide the proper basis for the highest reliability necessary for applying the teaching of qualitative interaction with respect to p53 in clinical practice.

In order to bring a biological marker to clinical use it is obligatory that the marker test provides highest sensitivity. Demonstration of highest sensitivity is an important issue for ethical approval as clinical decision making depends directly on the test result.

The p53 status determination according to the present invention requires highest sensitivity. This can only be achieved by the use of a sensitive sequencing technique with adequate primer positioning and with coverage of the essential regions of the p53 gene. Therefore, it is essential for the present invention that the PCR covers at least exon 2 to exon 11 of the p53 gene of the EMBL sequence U94788 (Seq.ID No. 1). Based on this information, suitable primers are disclosed in the prior art or providable by appropriate methods known to the person skilled in the art. In the example section, a specific primer set is disclosed which allows a superior testing in multiplex format. Accordingly, the region from bp 11619 to bp 18741, preferably the region from bp 11689 to bp 18680 is specifically preferred for PCR testing according to the present invention.

The feasibility of the test according to the present invention is also determined by the laboratory effort and the availability of the source material. The laboratory effort of sensitive sequencing can be markedly reduced by using the multiplex PCR approach according to the present invention. Surprisingly, this multiplex approach is possible in practice without risking significantly reduced reliability of the overall results of the testing. As a source material for the test, paraffin embedded tumour biopsies or specimen prepared during standard pathological work up can be used (besides samples directly taken from the patient). This is a major advantage compared to most chip based technologies testing gene expressions; the latter use RNA which requires deep frozen material. RNA harvesting from paraffin is questionable due to denaturation. Paraffin embedded tumour biopsies or specimen are routinely available as they are obligatory for tumour diagnosis.

With the method according to the present invention, reporting of the test result can be standardised. The test clearly indicates the result and avoids by its nature interobserver variability. Since the present test delivers a yes/no decision, this format is advantageous over those tests which need "manual" (microscopically) scoring (e.g. immunohistochemistry). The present sequencing test according to the present invention delivers a yes/no result (mutated or not) which avoids interobserver variability and discussion about the correct cut off level.

The application of different and mainly insensitive p53 analysis methods, aggravated by the lack of standards and reproducibility have been the major sources for the inconsistency of p53 research literature. This makes the selection of the quality controlled multiplex PCR plus sequencing approach according to the present invention superior against the other standard methods for determining p53 status of tumour patients:
Immunohistochemistry (IHC): In many studies p53 IHC has been used to screen for p53 alterations, because it is a very fast and easy method. However, the use of different tumour materials, technical conditions, different antibodies and scoring systems led to inconsistent results. Additionally there are many reasons for false positive and negative results which have been described (Wynford-Thomas, J. Pathol. 166 (1992), 329-330).

Activation results in stabilization of the p53 protein and allows its detection by IHC, but without further specification IHC is unable to differentiate between pathological accumulation due to gene mutation and physiological accumulation due to cellular stress. Physiological stabilization produces false positive IHC results. On the other hand, mutations at antibody binding sites targeted by IHC or the formation of a premature STOP codon by a gene mutation can prevent p53 detection by IHC completely and produce false negative results. Several studies confirmed this discrepancy between protein accumulation detected with IHC and the presence of a mutation in DNA sequencing (Kandioler et al., Ann. Surg. 235 (2002), 493-498; Karim et al., World J. Gastroenterol., 15 (2009), 1381-1387). Therefore most studies based on p53 IHC may not reflect the correct p53 status and conclusions drawn are questionable.

When data from the IARC TP53 somatic mutation database (Petitjean et al., Hum. Mutat. 28 (2007), 622-629)) concerning the type of mutation and the result of immunohistochemistry are evaluated, the results of this calculation can be summarised as follows (see also: Table 2). As it is very likely that frameshift mutations, large deletions, nonsense and splice site mutations lead to an impaired protein function, immunohistochemistry does not detect at least 12 % (919/7577) of non-functional types of mutations in the database.

**Table 2: Correlation between mutation type and IHC staining.**

| Gene mutation type | IHC negative (%) | IHC positive (%) | total |
|---|---|---|---|
| frameshift | 447 (66.6) | 224 (33.4) | 671 (8.8) |
| intronic | 24 (46.2) | 28 (53.8) | 52 (0.7) |
| large deletion | 2 (100) | 0 | 2 (0.03) |
| missense | 694 (11.9) | 5127 (88.1) | 5821 (76.8) |
| nonsense | 385 (71.0) | 157 (29.0) | 542 (7.2) |
| silent | 132 (36.7) | 228 (63.3) | 360 (4.8) |
| splice | 85 (65.9) | 44 (34.1) | 129 (1.7) |

It follows that IHC may produce false positive as well as false negative results. Additionally scoring systems for reporting IHC results are arbitrary and influenced by the observer and therefore not recommended for treatment decision making.

In contrast to IHC, any sequencing test delivers a yes/no result (mutated or not) which avoids interobserver variability and discussion about scoring systems.

Single-strand conformation polymorphism (SSCP): The analysis of SSCP to detect genetic variants is based on a sequence dependent migration of single-stranded DNA in polyacrylamide gel electrophoresis. A mutation in a known fragment is likely to lead to a conformational change of the DNA single-strand resulting in an aberration of migration characteristics. This method is fast and easy, but requires constant analysis conditions to deliver reproducible results. Furthermore fragments have to be rather small (max. 200 bp) to be sure that small changes (base exchanges, deletions or insertions of single bases) exert influence on the secondary structure.

Results from SSCP analysis may indicate the presence of a genetic variant, but they do not elucidate its nature. Therefore this method is frequently used as prescreening followed by sequencing of a fragment with an aberrant migration in gel electrophoresis. However, not all genetic variants lead to conformational changes so the absence of a positive SSCP result is not the proof for an intact gene.

Therefore, SSCP can be applied as a pre-screening method for mutation detection, but cannot replace DNA sequencing to identify the underlying mutation. In case of a negative SSCP result samples have to be sequenced anyway. On contrast to SSCP, the p53 test based on DNA sequencing according to the present invention not just indicates the presence of a mutation, but identifies its nature and allows a prediction of its impact on protein function.

Denaturing high-performance liquid chromatography (D-HPLC): PCR fragments are separated on a reverse-phase high performance liquid chromatography column under partially denaturising conditions and visualised in an UV-detector. DHPLC is based on DNA heteroduplex formation between wild-type and variant fragments, which can be separated from homoduplex molecules. It is described to be a very sensitive method and allows detection of low abundance variant alleles, but as SSCP it does not identify the nature of the variant.

SSCP, D-HPLC can be applied as a pre-screening method for mutation detection, but cannot replace DNA sequencing to identify the underlying mutation. In case of a negative D-HPLC result samples have to be sequenced anyway. From that point of view, SSCP and D-HPLC means additional laboratory effort and harbours a certain risk to miss mutations. Indeed, the p53 test according to the present invention does not just indicate the presence of a mutation, but identifies its nature and allows a prediction of its impact on protein function.

Mutation chip: The Amplichip p53 has been designed to detect single base-pair substitutions and single base-pair deletions in the coding sequence of the p53 gene. Currently the impossibility to identify insertions or deletions of more than one base-pair as well as intronic variants that impair splice-sites makes this method useless for clinical application to reliably detect p53 mutations.

Table 3 gives an overview of the number of mutations which can or cannot be detected with the chip.

**Table 3: Frequency of mutation types in the IARC-database; the mutations in the right column cannot be detected by the mutation chip.**

| Gene mutation type | Detected by chip (%) | Not detected by chip (%) |
|---|---|---|
| deletion | 111 (9.8) | 167 (14.7) |
| insertion | 0 (0) | 102 (9.0) |
| base exchange | 726 (64.0) | 0 (0) |
| intronic variants | 0 (0) | 27 (2.4) |
| complex frameshift | 0 (0) | 1 (0.1) |
| total | 838 (73.8) | 297 (26.2) |

The Amplichip p53 has been designed to detect a large proportion of p53 mutations. As calculated from the IARC Database and outlined in Table 3 about a quarter of mutations currently described cannot be detected with the chip. Compared to all other methods, especially also the chip based detection, the p53 test based on DNA sequencing according to the present invention provides highest sensitivity and specificity and is not limited in detecting any type of mutation.

Hot-spot sequencing (restriction to exons 5-8): The IARC p53 Mutation prevalence database (Petitjean et al., 2007) includes data from 91112 tumours published in 1485 references. As outlined in Table 4 most tumour mutation data are based on the analysis of exons 5 to 8. Exons 4 and 9 were analysed in only 50 % of the tumours published and exons 2, 3 and 11 in less than 20 %.
analysed 2 3 4 5 6 7 8 9 10 11 % 18.5 18.6 41.6 99.6 99.4 99.95 99.7 49.9 24.8 17.1 Mutation prevalence data
Table 4: Exons of the p53 gene which were analysed in published studies

In summary, 47 % of 2132 tumour collectives were analysed for exons 5 to 8 and showed an overall mean mutation rate of 32.1 %.
In 326 (15.3 %) collectives that were analysed for mutations in the whole gene (exons 2-11) the mean mutation rate was 37.2 %.

Since the p53 test according to the present invention analyses all exons of the gene including adjacent intronic regions to detect splice site mutations, the risk of not observing relevant mutations is excluded.

Sequencing of the whole p53 gene: Comparing mutation rates reported from studies which used a whole gene sequencing method to the data collected with the present invention, the reported rates are found to be lower. The reasons for that could be:
- inadequate primer positioning (introns, splice sites)
- bad sequencing conditions (high background masking mutations)
- lacking evaluation experience (missing of low abundance variant alleles)
- using of computer programs for sequencing-data-evaluation (programs are currently not ready for the detection of low abundance variant alleles)

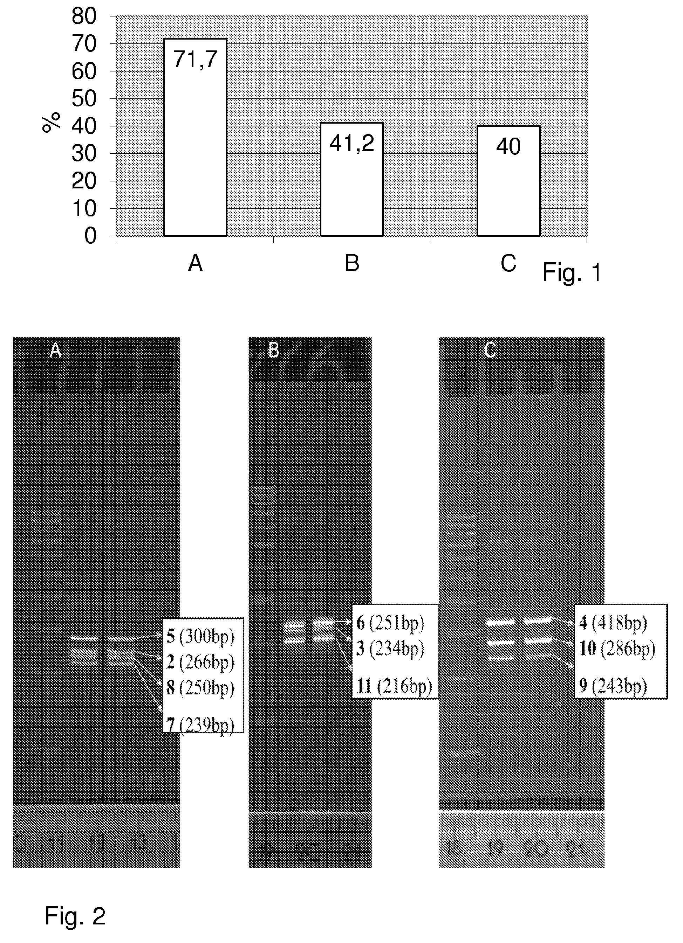

An interim evaluation of mutation rates of the trials conducted with the present invention (see also: example section) compared to the mutation rates of the IARC and the UMD database shows that the mean mutation rate is too low (even when only whole gene sequencing data are considered). As outlined in Figure 1 the mutation rate in the collective made with the present invention is markedly higher compared to published data compiled in databases.

The p53 test according to the present invention has been evaluated in a number of trials (see example section) and it could be consistently shown that the results obtained with the present method are of relevance for predicting cancer therapy response. Furthermore the test according to the present invention includes a number of quality control steps: PCR amplification products are visualised after polyacrylamide-gel electrophoresis to inspect amount, size and purity of the respective fragments and possible contaminations. All analyses are done in triplicates using forward end reverse primers for sequencing. Detection of sequence variants is always done by comparison of sequence curves from different samples to the reference sequence (Accession no.: U94788), e.g. by visual control.

For quality and quantity assessment PCR products are analysed on precast 5 % acrylamide/bisacrylamide gels (Criterion Gels, Bio-Rad Laboratories GmbH, Vienna, Austria). These gels have to be used with electrophoresis cells from the Criterion Precast Gel system and prepared according to the manufacturer's instructions. Before loading samples, each well of the gel has to be rinsed with 1x TBE (Tris/borate/EDTA: 0.1 M Tris, 0.09 M Boric Acid, and 0.001 M EDTA (Invitrogen, Paisley, UK)) running buffer. An aliquot (10 µl) of the reaction is mixed with 1 µl loading dye (Elchrom Scientific, Cham, Switzerland) and transferred into one well of the gel each. Similarly at least one well of each gel is used for a molecular weight marker (100 bp Molecular Ruler, Bio-Rad Laboratories GmbH, Vienna, Austria). Electrophoresis is performed at 130 V for 45 min followed by an ethidiumbromide staining (1 µg/ml; Bio-Rad Laboratories GmbH, Vienna, Austria) for 10 min. Bands of PCR products can be visualised on a transilluminator under UV-Light (Gel documentation system, Genxpress, Wiener Neudorf, Austria). Depending on the intensity of the respective bands 10-20 µl of PCR product is used for further analysis.

Upon visualization of the PCR products, one band for each amplified fragment of the respective size has to be visible. No additional smaller fragments in the range from 25 bp up to the smallest expected product are acceptable. Larger fragments in certain samples may arise due to certain mutations (insertions or deletions of a few bp). These fragments will not interfere with further analysis. In the negative control no band should be detected.

In case of lack of one or more expected products, smaller products as expected or any band in the negative control the whole PCR amplification (all samples amplified in parallel with the respective primer-mix) has to be withdrawn.

Although some of these steps might have been used by others in the prior art in an isolated manner, the provision of the quality assurance according to the present invention by applying these steps, preferably as a whole, is enabling the superior results of the present invention compared to other PCR/sequencing approaches for p53 in the prior art.

In summary, although p53 sequencing was a method which has been frequently applied in the prior art for detecting p53 mutations, it was only clear after performing or diligently considering each of the established methods that this is the most appropriate, most reliable and most practical method for performing the p53 status determination according to the present invention, provided that the quality-assuring steps of the present invention are carefully applied.

Other methods to investigate the p53 gene: Recently a clinical trial has been published using a yeast assay for the detection of p53 mutations with functional impact (Bonnefoi eta l., J. Clin. Oncol. 28 (2010), LBA503). Dysfunctional mutations were reported to be present in 43.8 % of breast cancer patients. Besides a problematical test reporting based on scoring system they failed to show an advantage for patients with dysfunctional p53 when treated with a combination therapy including a taxane. However, even if the method of the yeast assay was sensitive (which is unproven), they failed to consider the two pathway model in their trial design. The trial mentioned above combined drugs acting via different pathways which is - due to the qualitative interaction - not beneficial. The unawareness of the two pathway model and the qualitative interaction in clinical trials is a major source for confusing data (besides the lacking sensitivity of p53 tests).

An important aspect of the present invention is to demonstrate and preserve the power of the marker p53 by providing and claiming a highly sensitive testing method and therewith preserving the marker for clinical use and application in tumour patients.

A p53 mutation according to the present invention is defined as any mutation in the genetic set-up of the tumour cell which affects the primary amino acid sequence of p53 protein and decreases apoptosis induction activity of the p53. It follows that the p53 mutations according to the present invention are all mutations resulting in frameshifts and all deletions and insertions in the coding region. Moreover, all single base substitutions in the coding area which result in a change in primary amino acid sequence are p53 mutations according to the present invention as well as mutations in the regulating regions which cause loss or decreased expression of p53 in comparison to healthy tissue. Finally, all mutations affecting splice sites, thereby resulting in a p53 protein with different amino acid sequence, are also included.

Genetic polymorphisms, i.e. variants which are present in normal tissue too, and silent mutations, i.e. mutations which cause no change in the encoded amino acid sequence, are, of course, not defined as p53 mutations according to the present invention.

Examples for p53 mutations according to the present invention are disclosed e.g. in Kato et al., PNAS 100 (2003), 8424-8429. Other examples can be found in various databases for p53, such as the IARC (International Agency for Research on Cancer; somatic p53 mutations in neoplastic cells or tissues, including metastases or cells derived from such cells or tissue).

The results of DNA sequencing in the course of the present invention, which is currently the most reliable method for p53 mutation analysis, comprise changes of nucleotides. For evaluation of the functional consequences caused by mutations several approaches have been proposed in the scientific literature as outlined below.

Based on the location of the mutation and the predicted amino acid alterations two categories of p53 mutations have been defined by Poeta et al. (N. Engl. J. Med. 357 (2007), 2552-2561): Disruptive mutations are non-conservative changes of amino acids located inside the key DNA-binding domain (L2-L3 region), or all DNA sequence alterations that introduce a STOP codon resulting in disruption of p53 protein production; non-disruptive mutations are conservative changes of amino acids (replacement of an amino acid with another from the same polarity/charge category) or non-conservative mutations outside the L2-L3 region (excluding stop codons).

As reviewed by Joerger et al. (Oncogene 26 (2007), 2226-2242) p53 mutations lead to a variety of structural and energetic changes in the protein. Recently, using molecular modelling Carlsson et al. (FEBS J. 276 (2009), 4142-4155) proposed a stability measure of the mutated p53 structure to predict the severity of mutations. Taking structural features and sequence properties into account a classification into deleterious and non-deleterious mutations was performed.

The functional property of mutant p53 proteins may also be represented by their transactivation activities (TAs), as measured in eight p53 response elements in yeast assays by Kato et al. (2003) and expressed as a percentage of wildtype protein.

The TAs for all possible missense mutations obtained by single-nucleotide substitution along the full coding sequence of p53 are listed in the database of the International Agency for Research on Cancer (Petitjean et al., Hum. Mutat. 28 (2007), 622-628). Perrone et al proposed the median TAs to be calculated and mutations to be classified as fully functional (median TA > 75% and ≤ 140%), partially functional (median TA > 20% and ≤ 75%), or non-functional (median TA ≤ 20%) (Perrone et al., J.Clin. Oncol. 28 (2010), 761-766).

All these approaches are based on the detection of nucleotide changes (mutations) brought together with general knowledge of protein expression (mechanisms of translation) as well as the functional domains of the protein. Thereby the impact on the function of the protein can be deduced. A base exchange at a certain position may create a stop codon, lead to the usage of another amino acid at this position or produce no apparent alteration. These changes happen at the level of translation, but a base exchange may be already effective in mRNA processing. A translationally silent mutation may produce or disrupt a splice site as well as a binding site for regulatory factors (proteins, microRNAs). On the other hand, even in case of an amino-acid exchange a protein may retain some of its normal functions.

Based on these arguments all mutations qualify as functionally relevant in some way unless there is a comprehensive scientific proof of normal function in-vivo.

Preferred p53 mutations to be detected according to the present invention are all mutations in the p53 gene, especially
- all mutations resulting in frameshifts
- all deletions and insertions in the coding region
- all single base substitutions in the coding area which result in a stop codon (nonsense mutations)
- all single base substitutions proven to affect splice sites in vivo or in vitro

Of the single base substitutions resulting in a change of the primary amino acid sequence or potentially affecting splice sites the following have been directly tested for interaction with chemotherapy in clinical studies:

**Table A**

| p53 Mutation | Pathway |
|---|---|
| c.313G>T (p.Gly105Cys) | 1 |
| c.332T>A (p.Leu111Gln) | 2 |
| c.380C>T (p.Ser¨127Phe) | 1 |
| c.422G>A (p.Cys141Tyr) | 1 |
| c.452C>G (p.Pro151Arg) | 1 |
| c.461G>T (p.Gly154Val) | 1 |
| c.467G>C (p.Arg156Pro) | 2 |
| c.473G>A (p.Arg158His) | 1 |
| c.488A>G (p.Tyr163Cys) | 1 |
| c.524G>A (p.Arg175His) | 1/ 2 |
| c.578A>T (p.His193Leu) | 1 |
| c.584T>C (p.Ile195Thr) | 1 |
| c.641A>G (p.His214Arg) | 2 |
| c.653T>A (p.Val218Glu) | 1 |
| c.659A>G (p.Tyr220Cys) | 1 |
| c.707A>G (p.Tyr236Cys) | 1 |
| c.711G>C (p.Met237Ile) | 1 |
| c.733G>A (p.Gly245Ser) | 1/ 2 |
| c.742C>T (p.Arg248Trp) | 1 |
| c.743G>A (p.Arg248Gln) | 1 |
| c.743G>T (p.Arg248Leu) | 1 |
| c.746G>T (p.Arg249Met) | 1 |
| c.747G>T (p.Arg249Ser) | 1 |
| c.749C>T (p.Pro250Leu) | 2 |
| c.785G>T (p.Gly262Val) | 1 |
| c.794T>C (p.Leu265Pro) | 1 |
| c.811G>A (p.Glu271Lys) | 1 |
| c.817C>T (p.Arg273Cys) | 1 |
| c.818G>A (p.Arg273His) | 1 |
| c.818G>T (p.Arg273Leu) | 1 |
| c.821T>C (p.Val274Ala) | 1 |
| c.824G>A (p.Cys275Tyr) | 1 |
| c.827C>A (p.Ala276Asp) | 1 |
| c.833C>G (p.Pro278Arg) | 1 |
| c.833C>T (p.Pro278Leu) | 1 |
| c.844C>T (p.Arg282Trp) | 1/ 2 |
| c.1025G>C (p.Arg342Pro) | 1 |
| c.919+1G>T | 2 |
| c.994-1G>A | 1 |

Table A: Single base substitutions detected in tumours and evaluated for interaction with cancer therapy according to pathway 1 (apoptosis induction) or pathway 2 (cell-cycle interference). For patients with tumours bearing one of the mutations listed, the therapy regimen acting in pathway 1 were not effective, those in pathway 2 led to improved outcome.

If any of these mutations occur in the analysis of the tumour cells according to the present invention, the p53 status is "mutated". Preferably, the presence/absence of the mutations according to Table A are investigated by the method according to the present invention (these can also be tested for a tumour already diagnosed in principle).

In summary, currently the issue of functional evaluation of p53 mutations has not been finally addressed, the proposed classifications arose from basic research approaches (partly in vitro) and are not proven in appropriately designed clinical trials.

Appropriately designed clinical trials currently do not exist because the clinical evaluation of the utility of p53 mutations deserves a number of considerations listed as follows:
1. The treatments used in such trials have to be synergistic considering their pathway of action or have to be single drugs (monotherapy).
   The qualitative interaction and the two pathway model has not yet been realised. A combination of treatments from different pathways - which is very common today- will bias the trial results. Consequently appropriate clinical trials currently do not exist.
2. The selection of the appropriate patient population and the choice of an adequately measurable end point.
   Ideally, the population studied should be one in which the knowledge of the marker would have substantial clinical relevance (e.g. tumour type with a high frequency of p53 mutations) and where the feasibility of obtaining appropriate specimens is established.
3. The adequate endpoint in a trial testing for therapeutic interaction is response to treatment. Measurement of response to treatment requires a clinical setting which allows a correct (pathological) response assessment. Pathological response assessment is available only for patients who are treated having their tumours in place. (i.e. preoperatively (neoadjuvantly) treated patients or patients treated for metastases).
4. The use of qualified statistical designs to allow statistical test for interaction. The latter requires specification of subsets (p53 normal, p53 mutant) in advance. For identification of the subsets (p53 normal, p53 mutants) a reliable method has to be used.
5. The issues listed above cannot be fulfilled retrospectively and therefore an evaluation of the clinical utility of p53 as a predictive marker cannot be done retrospectively. Retrospective evaluation can reach the evidence of hypothesis finding studies and/or classify a marker as promising.

Based on the above listed considerations the first prospective randomised clinical trial qualified to evaluate the importance of the functionality of p53 mutations was initiated (see "PANCHO trial" in the example section). This clinical trial was conducted by using the method according to the present invention and has consistently supported the reliability of the present method.

The data according to the present invention consistently showed that p53 sequencing completely demarcated the group of non-responders supporting that "all mutations have functional impact" teaching.

The invention is further described in the following examples and the drawing figures, yet without being restricted thereto.
Figure 1 shows p53 mutation rate in oesophageal cancer: Percentage of mutated tumours reported by (A) p53 Research, (B) IARC p53 Database, (C) UMD p53 Database;
Figure 2 shows gelelectrophoresis of multiplex PCR products: (A) Exons 5, 2, 8 and 7 are amplified with primermix M1, (B) Exons 6, 3 and 11 with primermix M2, (C) Exons 4, 10 and 9 with primermix M3. Fragment sizes are specified in basepairs (bp);
Figure 3 shows the sequencing data of samples 2234 and 2235; (A) Forward sequencing: Both mutations are visible (hatched and dotted arrow); (B) Reverse sequencing; mutation sample 2234 barely visible, but the peak height is lower than normal as shown in sample 2235 (hatched arrow), mutation sample 2235 visible (dotted arrow); all mutated positions show a lower height of the normal peak compared to the neighbouring peaks and the sequence without a mutation;
Figure 4 shows the primers used in the examples section of the present invention in comparison with the IARC primers;

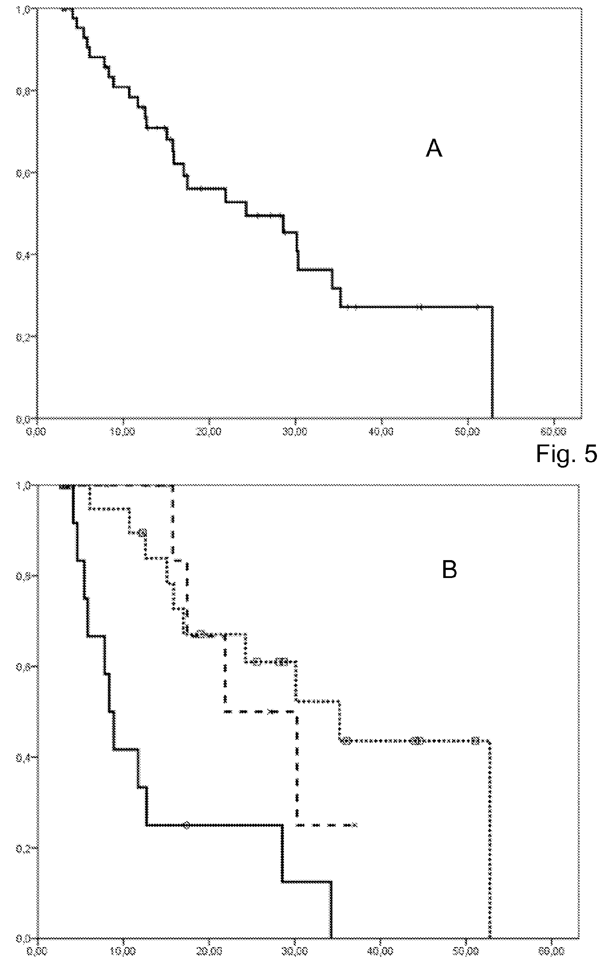

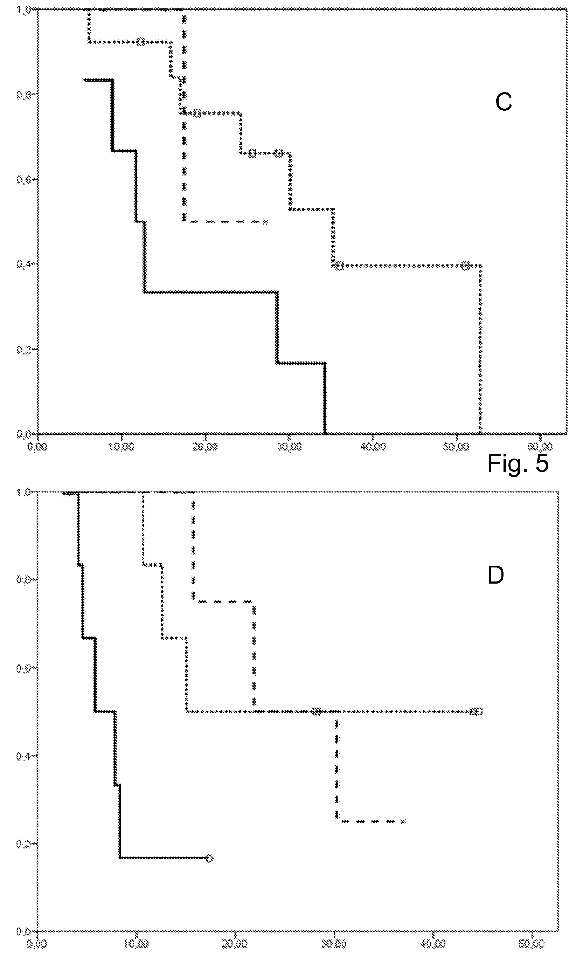

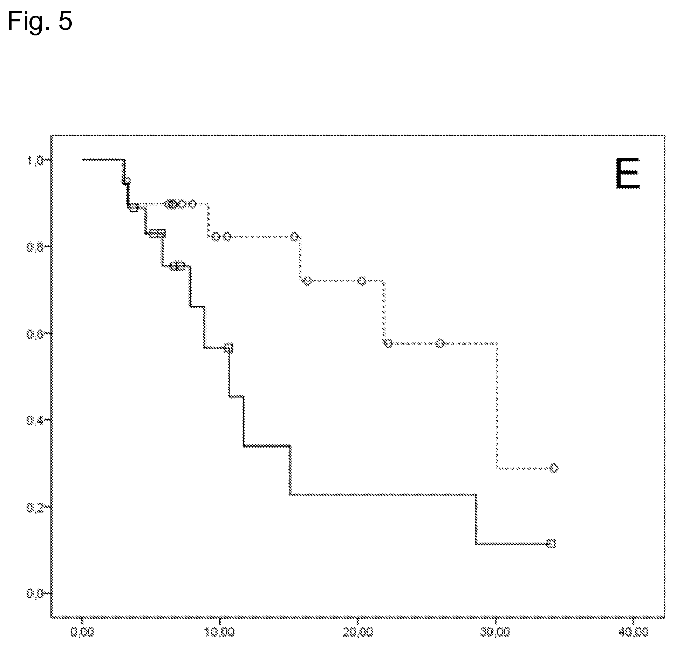

Figure 5: Data from Key Study: Oesophageal cancer pilot study: (A) Overall survival of neoadjuvantly treated oesophageal cancer patients (n=47); treatment was either Cisplatin/5-FU (n=36) or Docetaxel (n=11); mean survival rate was 24.2 months; (B) survival of patients with p53 normal tumours and Cis/5-FU treatment (dotted line; mean survival 34 months); p53 mutated tumours and Cis/5-FU treatment (full line; mean survival 14 months); p53 mutated tumours and Docetaxel treatment (dashed line; mean survival 26 months) ; p<0,001; (C) line shapes are analogous to (B); mean survival of patients with adenocarcinoma (n=24) was 35 (dotted line) vs. 17 (full line) vs. 22 months (dashed line); p=0.024; (D) line shapes are analogous to (B); mean survival of patients with squamous cell carcinoma (n=23) was 26 (dotted line) vs. 8 (full line) vs. 29 months (dashed line); p=0.01; (E) overall survival of patients treated with p53 adjusted therapy (dotted line; mean survival 30 months) and p53 non adjusted therapy (full line; mean survival 15 months); p=0.042;

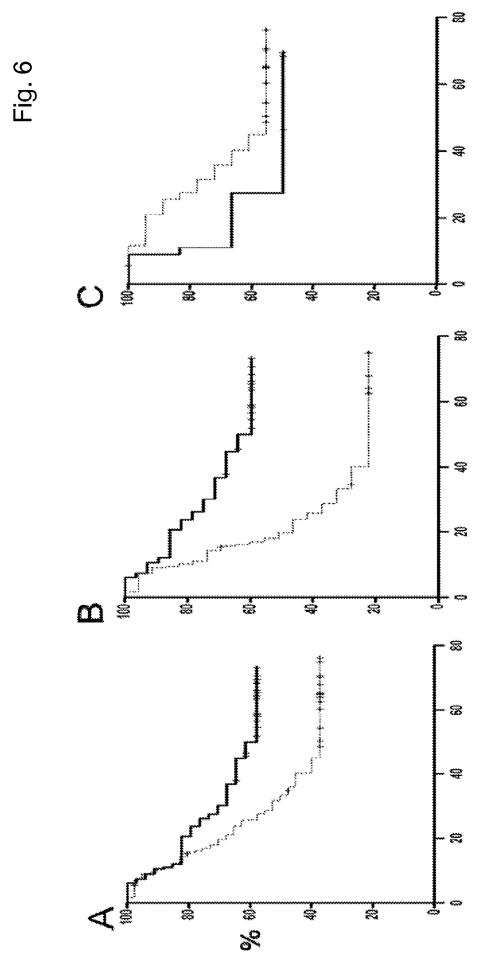

Figure 6 shows data from Key Study: CRCLM (colorectal cancer liver metastases) : full line: p53 normal; dotted line : p53 mutant; (A) survival of patients with CRCLM (n=76) with normal p53 (full line) and mutant p53 (dotted line); (B) subset of patients treated with preoperative chemotherapy 5-FU/Oxaliplatin (n=51) with normal p53 (full line) and mutant p53 (dotted line); p=0.025; Cox-model was used to calculate Hazard ratio 3.24 (95%CI 1.5-7.0); p=0.045; adjustment to known prognostic parameters (age, sex, T-stage, N-stage, grading, synchronous/metachronous tumours), results in a hazard ratio of 5.491 (95%CI 2.28-13.24); p=0.0042; (C) subset of patients without preoperative Chemotherapy (no chemotherapy); normal p53 (full line) is not related to improved survival; p=0.543; patients receiving preoperative chemotherapy show better survival than those receiving no chemotherapy in case they have a normal p53 gene (full lines (B) and (C)); patients receiving preoperative chemotherapy do worse than patients receiving no chemotherapy in case they have a p53 mutation (dotted lines (B) and (C)); patients with p53 mutated tumours receiving 5-FU/oxaliplatin chemotherapy have a 5.49 fold risk to die (p=0.042);
Figure 7 shows marker by treatment interaction design to test a predictive factor question: Sargent et al., J. Clin. Oncol. 23 (2005), 2020-2027;
Figure 8 shows cumulatively reported number of mutations in the years 1985 to 2008: Full line - all mutations, line with squares - all new mutations, line with triangles - new missense mutations;

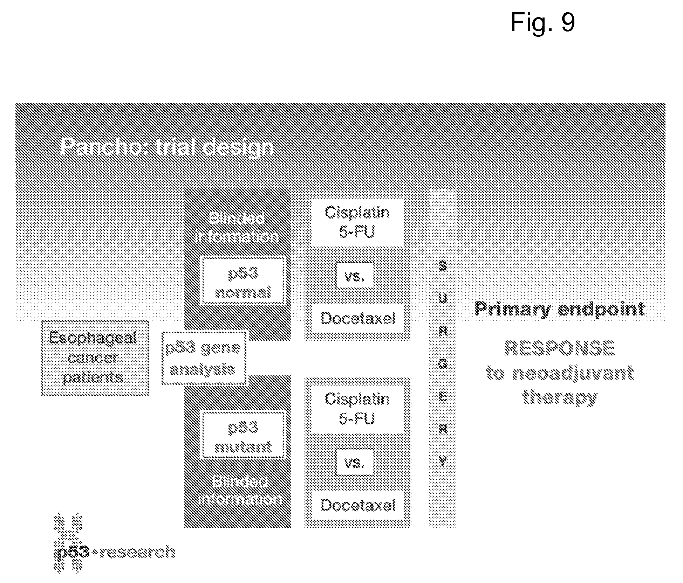

Figure 9 shows PANCHO: the trial design: Eligible for the PANCHO trial are operable oesophageal cancer patients >T1 stage. P53 gene analysis is performed as marker test. Patients are stratified for their histological type (adeno-, squamous cell carcinoma); marker negative patients (p53 normal) are randomised to receive either Cispaltin/5-FU or Docetaxel preoperatively; marker positive patients are also randomized to receive either Cisplatin/5-FU or Docetaxel; after three cycles of preoperative chemotherapy all patients are referred to surgery; response to neoadjuvant therapy is defined as primary endpoint and is assessed comparing the diagnostic tumour stage with the pathological tumour stage.

### EXAMPLES:

### I. p53 test according to the present invention

### 1. Background

The p53 gene is located on the short arm of chromosome 17 in the region 17p13.1. The genomic region spans approximately 22 kb where the coding sequence is arranged in 11 exons. Start of the translation for the 2.2 kb mRNA is in exon 2 with the first nucleotide at position 11717 and the last nucleotide at position 18680 (exon 11) of the sequence with the accession number U94788 (Seq.ID.No.1). Detailed information on the size and location of exonic and intronic regions according to the published sequence is given in Table 5.

**Table 5: p53 - exons and introns**

| | number | size (bp) | nt start¹ | nt end¹ |
|---|---|---|---|---|
| exon | 2 | 102 | 11689 | 11790 |
| exon | 3 | 22 | 11906 | 11927 |
| exon | 4 | 279 | 12021 | 12299 |
| exon | 5 | 184 | 13055 | 13238 |
| exon | 6 | 113 | 13020 | 13432 |
| exon | 7 | 110 | 14000 | 14109 |
| exon | 8 | 137 | 14452 | 14588 |
| exon | 9 | 74 | 14684 | 14754 |
| exon | 10 | 107 | 17572 | 17678 |
| exon | 11 | 82 | 18599 | 18680 |
| intron | 2 | 115 | 11791 | 11905 |
| intron | 3 | 93 | 11928 | 12020 |
| intron | 4 | 755 | 12300 | 13054 |
| intron | 5 | 81 | 13239 | 13319 |
| intron | 6 | 567 | 13433 | 13999 |
| intron | 7 | 342 | 14110 | 14451 |
| intron | 8 | 92 | 14589 | 14680 |
| intron | 9 | 2817 | 14755 | 17571 |
| intron | 10 | 920 | 17679 | 18598 |

| | | | | |
|---|---|---|---|---|
| ¹Nucleotide position according to sequence U94788 | | | | |

### 2. Primer design for multiplex PCR

Primers were designed with the Primer3 software package (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386). Melting temperature of primers was set to range from 65 to 68 °C and there should preferably be a distance of at least 30 bp between primers and exon sequence. The melting temperature depends on the DNA sequence of the primer region and has to be lower than 72°C, which is the optimal temperature for most polymerases used for PCR amplification. At the same time melting temperature has to be as high as possible to prevent amplification of products outside of the region of interest where a primer has bound only partially. A uniform annealing temperature of all primer-pairs used for p53 amplification allows simultaneous amplification of several fragments in a single reaction. The distance between primer position and exon sequence is essential to guarantee analysis of the whole coding sequence including splice sites at the intron-exon-borders.

Furthermore to allow sequence analysis of DNA fragments amplified simultaneously in one reaction primer-binding sites must not lead to overlapping amplicons.

Detailed information on the amplicons and primer sequences is given in Table 6.

**Table 6: p53 - amplicons and primers for PCR**

| | number | size (bp) | nt start1 | nt end1 | sequence |
|---|---|---|---|---|---|
| amplicon | 2 | 250 (266) | 11619 | 11868 | |
| amplicon | 3 | 234 | 11833 | 12066 | |
| amplicon | 4 | 418 | 11937 | 12354 | |
| amplicon | 5 | 300 | 12991 | 13290 | |
| amplicon | 6 | 251 | 13245 | 13495 | |
| amplicon | 7 | 239 | 13926 | 14164 | |
| amplicon | 8 | 250 | 14391 | 14640 | |
| amplicon | 9 | 243 | 14608 | 14850 | |
| amplicon | 10 | 286 | 17464 | 17749 | |
| amplicon | 11 | 216 | 18526 | 18741 | |
| primer f | 2 | 21 | 11619 | 11638 | (gatcgatcgatcgatc) |
| | | | ttctctgcaggcccaggtga (Seq.ID.No.2/3) | | |
| primer r (Seq.ID.No.4) | 2 | 21 | 11848 | 11868 | tcgcttcccacaggtctctgc |
| primer f (Seq.ID.No.5) | 3 | 20 | 11833 | 11852 | aaccccagccccctagcaga |
| primer r (Seq.ID.No.6) | 3 | 20 | 12047 | 12066 | ccggggacagcatcaaatca |
| primer f (Seq.ID.No.7) | 4 | 19 | 11937 | 11955 | agggttgggctggggacct |
| primer r (Seq.ID.No.8) | 4 | 21 | 12334 | 12354 | gggatacggccaggcattga |
| primer f (Seq.ID.No.9) | 5 | 29 | 12991 | 13017 | ccagttgctttatctgttcacttgtgc |
| primer r (Seq.ID.No.10) | 5 | 18 | 13273 | 13290 | ctggggaccctgggcaac |
| primer f (Seq.ID.No.11) | 6 | 20 | 13245 | 13264 | agctggggctggagagacga |
| primer r (Seq.ID.No.12) | 6 | 19 | 13477 | 13495 | ccggagggccactgacaac |
| primer f (Seq.ID.No.13) | 7 | 20 | 13926 | 13945 | aaaaggcctcccctgcttgc |
| primer r (Seq.ID.No.14) | 7 | 19 | 14146 | 14164 | aagcagaggctggggcaca |
| primer f (Seq.ID.No.15) | 8 | 24 | 14391 | 14414 | tgggacaggtaggacctgatttcc |
| primer r (Seq.ID.No.16) | 8 | 23 | 14618 | 14640 | ggcataactgcacccttggtctc |
| primer f (Seq.ID.No.17) | 9 | 20 | 14608 | 14627 | agcggtggaggagaccaagg |
| primer r (Seq.ID.No.18) | 9 | 22 | 14829 | 14850 | tgccccaattgcaggtaaaaca |
| primer f (Seq.ID.No.19) | 10 | 23 | 17464 | 17486 | tcgatgttgcttttgatccgtca |
| primer r (Seq.ID.No.20) | 10 | 25 | 17725 | 17749 | aatggaatcctatggctttccaacc |
| primer f (Seq.ID.No.21) | 11 | 20 | 18526 | 18545 | ggtcagggaaaaggggcaca |
| primer r (Seq.ID.No.22) | 11 | 20 | 18722 | 18741 | tggcaggggagggagagatg |
| primer seq f (Seq.ID.No.23) | 4 | 19 | 11992 | 12010 | ctctgactgctcttttcac |
| primer seq r (Seq.ID.No.24) | 4 | 20 | 12321 | 12340 | cattgaagtctcatggaagc |

| | | | | | |
|---|---|---|---|---|---|
| Nucleotide position according to sequence U94788; f forward, r reverse; primer f 2 contains a 4 x gate elongation; this is a preferred embodiment to provide a better distinguishability between exons 2 and 8 in mix 1 without influence on primer binding an reaction conditions. | | | | | |

### 3. Multiplex PCR amplification

With the primers listed in Table 6 all coding exons of the p53 gene can be amplified in 3 PCR reactions followed by individual sequence analyses. Because of a repetitive sequence in intron 3 which had to be included in the amplicon due to proximity to the start of the exon, another forward primer had to be chosen for sequence analysis of exon 4. The reverse primer for exon 4 sequencing also differs from that used for PCR amplification, as it gave improved results - which were shown in stronger signals and less background. All other exons can be sequenced with the same primers used for PCR amplification.

The forward primer of exon 2 was elongated at the 5-prime end by a non-complementary fragment of 4 GATC-series to give a distinguishable band in polymerase gel electrophoresis. This allows a quality test for each amplification reaction.

All PCR-amplifications are optimised to be performed in a Biometra Thermocycler T1 or T-gradient (Biometra, Göttingen, Germany).

### 3.1 p53 amplification mix M1

In the mix M1 exons 2, 5, 7 and 8 are amplified simultaneously in one reaction. Primers are stored in stock solutions of 100 µM. A working solution containing the respective concentration ratio is prepared from 10 µM solutions; 2.6 µl of the working solution are added to each amplification reaction.

**Table 7: Composition of the PCR-reaction for mix M1**

| amount¹ | substance | supplier²/cat-number |
|---|---|---|
| to a total volume of 50 µl | pure water | Merck/116434 |
| 6 µl | Buffer I | Applied Biosystems/N8080244 |
| 250 µM | dNTP | Applied Biosystems |
| 2,5 U | Ampli Taq | Applied Biosystems/N8080244 |
| | Gold Polymerase | |
| 80 µM | primer 2f | Sigma-Genosys |
| 80 µM | primer 2r | Sigma-Genosys |
| 60 µM | primer 5f | Sigma-Genosys |
| 60 µM | primer 5r | Sigma-Genosys |
| 60 µM | primer 7f | Sigma-Genosys |
| 60 µM | primer 7r | Sigma-Genosys |
| 60 µM | primer 8f | Sigma-Genosys |
| 60 µM | primer 8r | Sigma-Genosys |
| 4 µl | sample DNA | |

| | | |
|---|---|---|
| ¹ Reaction volume is 50 µl ² Applied Biosystems, Foster City, CA; Merck, Darmstadt, Germany; Sigma-Aldrich, Vienna, Austria. | | |

**Table 8: Cycling protocol for mix 1**

| step | temperature (°C) | time (s) | ramp (°C/s) | no of cycles¹ |
|---|---|---|---|---|
| 1 | 95 | 600 | 5 | |
| 2 | 95 | 40 | 5 | |
| 3 | 64 | 40 | 5 | 47 |
| 4 | 75 | 60 | 3 | |
| 5 | 72 | 600 | 5 | |
| 6 | 15 | hold | 5 | |

| | | | | |
|---|---|---|---|---|
| ¹ One cycle include steps 2 to 4 which are repeated the respective times | | | | |

### 3.2 p53 amplification mix M2

In the mix M2 exons 3, 6 and 11 are amplified simultaneously in one reaction. Primers are stored in stock solutions of 100 µM. A working solution containing the respective concentration ratio is prepared from 10 µM solutions; 2.4 µl of the working solution are added to each amplification reaction.

**Table 9: Composition of the PCR-reaction for mix M2**

| amount¹ | substance | supplier²/cat-number |
|---|---|---|
| to a total volume of 30 µl | pure water | Merck/116434 |
| 15 µl | QIAGEN® Multiplex | Qiagen/206145 |
| | PCR Kit | |
| 40 µM | primer 3f | Sigma-Genosys |
| 40 µM | primer 3r | Sigma-Genosys |
| 80 µM | primer 6f | Sigma-Genosys |
| 80 µM | primer 6r | Sigma-Genosys |
| 120 µM | primer 11f | Sigma-Genosys |
| 120 µM | primer 11r | Sigma-Genosys |
| 4 µl | sample DNA | |

| | | |
|---|---|---|
| ¹ Reaction volume is 30 µl ² Merck, Darmstadt, Germany; Qiagen, Hilden, Germany; Sigma-Aldrich, Vienna, Austria. | | |

**Table 10: Cycling protocol for mix 2.**

| | temperature (°C) | time (s) | ramp (°C/s) | no of cycles¹ |
|---|---|---|---|---|
| 1 | 95 | 600 | 5 | |
| 2 | 95 | 40 | 5 | |
| 3 | 64 | 90 | 5 | 47 |
| 4 | 76 | 40 | 3 | |
| 5 | 72 | 600 | 5 | |
| 6 | 15 | hold | 5 | |

| | | | | |
|---|---|---|---|---|
| ¹ One cycle include steps 2 to 4 which are repeated the respective times | | | | |

### 3.3 p53 amplification mix M3

In the mix M3 exons 4, 9 and 10 are amplified simultaneously in one reaction. Primers are stored in stock solutions of 100 µM. A working solution containing the respective concentration ratio is prepared from 10 µM solutions; 2.3 µl of the working solution are added to each amplification reaction.

**Table 11: Composition of the PCR-reaction for mix M3.**

| amount¹ | substance | supplier²/cat-number |
|---|---|---|
| to a total volume of 50 µl | pure water | Merck 116434 |
| 25 µl | QIAGEN® Multiplex | Qiagen/206145 |
| | PCR Kit | |
| 100 µM | primer 4f | Sigma-Genosys |
| 100 µM | primer 4r | Sigma-Genosys |
| 30 µM | primer 9f | Sigma-Genosys |
| 30 µM | primer 9r | Sigma-Genosys |
| 100 µM | primer 10f | Sigma-Genosys |
| 100 µM | primer 10r | Sigma-Genosys |
| 4 µl | sample DNA | |

| | | |
|---|---|---|
| ¹ Reaction volume is 50 µl ² Merck, Darmstadt, Germany; Qiagen, Hilden, Germany; Sigma-Aldrich, Vienna, Austria. | | |

**Table 12: Cycling protocol for mix 3.**

| | temperature (°C) | time (s) | ramp (°C/s) | no of cycles¹ |
|---|---|---|---|---|
| 1 | 95 | 600 | 5 | |
| 2 | 95 | 40 | 5 | |
| 3 | 65-1/cycle² | 90 | 5 | 5 |
| 4 | 75 | 60 | 3 | |
| 5 | 95 | 40 | 5 | |
| 6 | 60 | 90 | 5 | 42 |
| 7 | 75 | 60 | 3 | |
| 8 | 72 | 600 | 5 | |
| 9 | 15 | hold | 5 | |

| | | | | |
|---|---|---|---|---|
| ¹ One cycle include steps 2 to 4 and afterwards steps 5 to 7 which are repeated the respective times ² Annealing temperature of the first cycle is 65 °C, in each of the following cycles the temperature is lowered for 1° | | | | |

### 4. PCR amplification quality control

For quality and quantity assessment PCR products are analyzed on precast 5 % acrylamide/bisacrylamide gels (Criterion Gels, Bio-Rad Laboratories GmbH, Vienna, Austria). An aliquot (10 µl) of the reaction is mixed with 1 µl loading dye (Elchrom Scientific, Cham, Switzerland) and transferred into one well of the gel each. Similarly at least one well of each gel is used for a molecular weight marker (100 bp Molecular Ruler, Bio-Rad Laboratories GmbH, Vienna, Austria). Electrophoresis is performed at 130 V for 45 min followed by an ethidiumbromide staining (1 µg/ml; Bio-Rad Laboratories GmbH, Vienna, Austria) for 10 min. Bands of PCR products can be visualised on a transilluminator under UV-Light (Gel documentation system, Genxpress, Wiener Neudorf, Austria; see Figure 2). Depending on the intensity of the respective bands 10-20 µl of PCR product is used for further analysis.

### 5. Purification of PCR products

To remove excess primers and dNTPs, 10-20 µl of each PCR product are purified with the illustra GFX™ PCR DNA and Gel Band Purification Kit (GE Healthcare, Munich, Germany).

### 6. Sequence analysis

For sequence analysis the BigDye Terminator Cycle Sequencing Kit (Applied Biosystems, Foster City, CA) is used according to the manufacturer's instructions. The reaction volume is 5 µl containing 1 µl Reaction Mix, 0.5 to 1 µl sample and 2 pmol primer. The standard cycling profile is applied - 25x (96°C 10s, 50°C 5s, 60°C 180s). Separate reactions have to be set up for each primer used. Excess dye-labelled terminators are removed using Centri-Sep spin columns (Applied Biosystems, Foster City, CA). Briefly, the column gel is hydrated with pure water (Merck, Darmstadt, Germany) at room temperature for 2 hours and spun in a microcentifuge at 750g for 2 min to remove the interstitial fluid. The sample is mixed with 15 µl pure water (Merck, Darmstadt, Germany), applied to the column and spun again. The filtrate is added to 20 µl Hi-Di™ formamide (Applied Biosystems, Foster City, CA) for loading to the instrument. Separation and analysis of the sequencing reaction products are performed on an ABI Prism^{®} 310 Genetic Analyzer or an Applied Biosystems 3130 Genetic Analyzer using standard protocols (see Table 13).

**Table 13: Capillary electrophoresis**

| Instrument | capillary length | polymer type | program |
|---|---|---|---|
| 310 | 47 cm | POP6 | ABI Prism™ 310 Collection v1.2.2 |
| 3130 | 50 cm | POP6 | 3130 Data Collection v3.0 |

| | | | |
|---|---|---|---|
| Applied Biosystems, Foster City, CA | | | |

### 7. Detection of sequence variants (mutations)

Sequence curves obtained from the analysis of different samples are aligned with the Autoassembler v2.1 or SeqScape v2.6 program (Applied Biosystems, Foster City, CA) and visually compared by a trained person. Sequence variants are detected by the appearance of more than one peak at one position in one sample compared to others as well as to the reference sequence (Accession no.: U94788).

### 8. Quality control issues

### 8.1 Quality and purity of PCR amplification products

Each PCR reaction setup is used without DNA to detect possible contaminations in any reagent used. To detect contaminations and to inspect amount and size of the respective fragments PCR amplification products are visualised after polyacrlyamid-gel electrophoresis.

### 8.2 Method for detection of mutations

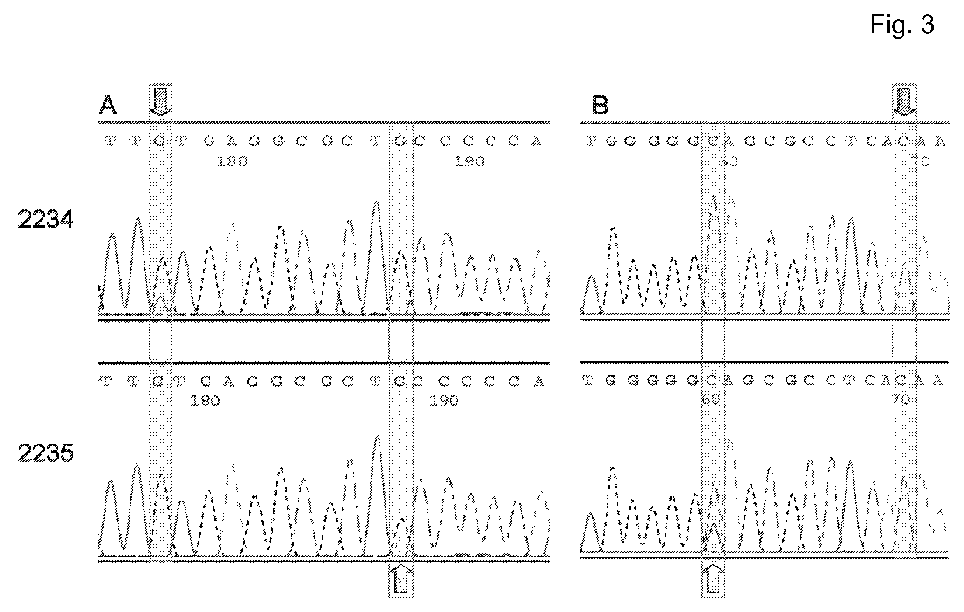

Visual inspection of sequence curves by an experienced person is currently the most reliable method to detect DNA variants. Visual detection of sequence variants is always done by comparison of sequence curves from different samples to the reference sequence (Accession no.: U94788). Results are confirmed by sequencing using forward and reverse primers after PCR amplification in triplicates. The impact of evaluating sequencing curves of both strands has recently been shown by Li et al. (Hum. Mutat. 30 (2009), 1583-1590). The mutation displayed in the supplemental figure S10 of this article (corresponds to Fig. 3 herein) is only visible in the forward, but not in the reverse strand. However, as no sequence curves of other samples were given in this publication, it could not be ruled out that a reduced height of the respective normal peak would have indicated the mutation, as shown in Fig. 3 (B) herein where the mutation in sample 2234 is barely visible, but the peak height is lower than normal as shown in sample 2235 (hatched arrow).

### 9. Performance of the method according to the present invention:

To evaluate several samples of a study sequencing traces from forward and reverse strand sequencing of two or more samples and the respective parts of the published reference sequence with the accession number U94788 are aligned as outlined in Fig. 3 (the maximum number of samples that can be evaluated simultaneously depends on the programme used and on the performance of the computer). Sequence curves are visually inspected to detect differences in the peak pattern. Samples can mutually serve as normal controls as long as they have mutations at different sites. Differences in the peak pattern are described according to the nomenclature for the description of sequence variants (http://www.hgvs.org/mutnomen/; den Dunnen & Antonarakis, Human Mutation 15 (2000) 7-12). In the manner specified all sequences from the triplicate PCR amplifications generated by forward and reverse strand sequencing of each exon are evaluated. This evaluation results in the classification of each sample as normal, if no difference to the reference sequence is detected, or mutated in case of a difference which is not listed as polymorphism e.g. in the IARC p53 database (http://www-p53.iarc.fr).

### 10. General remarks

The advantages of the method according to the present invention are specifically pronounced when the test is applied in connection with the p53 qualitative interaction. These advantages are due to the combination of a quality-controlled, triplicate multiplex PCR as disclosed herein and the automated sequencing using forward and reverse primers for sequencing for all amplicons of the triplicate set-up and, preferably, the visual inspection of the sequence.

Already the primer design in the PCR according to the present invention is diligently performed. The primer set as disclosed in the example section of the present application have provided strikingly good and reliable results. However, when respecting general rules of primer design, primers can be also positioned differently from the position selected in the present examples, but the following issues have to be considered:
- the amplicon must include the whole exon including splice sites
- annealing temperature has to be similar for primers intended to be multiplexed
- a multiplex reaction must not include overlapping amplicons
- amplicons of a multiplex reaction must differ in size to allow quality check by gel electrophoresis
- amplicons should be as short as possible to allow amplification from difficult samples with a high degree of DNA degradation (e.g. formalin-fixed, paraffinised tissues)

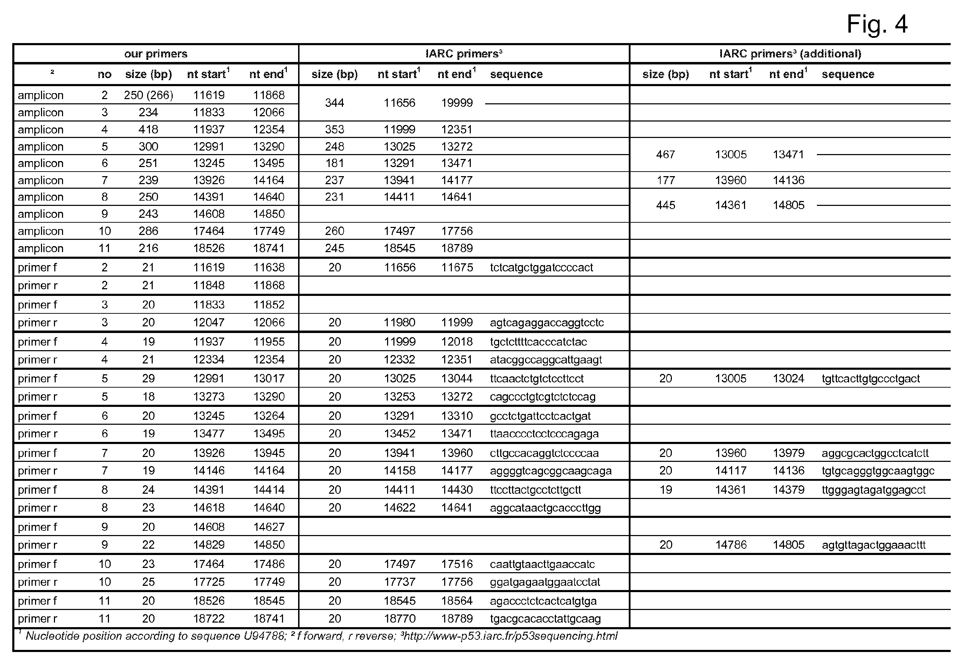

A comparison of primers used in the present example section to those used by the IARC sequencing service is given in Fig. 4. Generally most primers from the IARC are located closer to the exon sequence than those of the present examples. Exons three and nine are analysed together with the respective preceding exon which results in large amplicons and may lead to insufficient amplification from difficult DNA samples.

As an example, primer design of exon 4 is shortly described: Intron 3 is quite short (115 bp) and contains a repetitive sequence stretch which impairs selection of a position for the forward primer. Most groups as well as the IARC selected a primer position close to the exon sequence (IARC uses 2 bp distance).

Due to technical reasons of DNA sequencing using the Sanger method, the first 5-20 bp after the primer cannot be evaluated with sufficient reliability for mutation detection. Especially when using the same primer for PCR amplification and sequencing this limitation is eminent. To circumvent this problem a PCR primer located adjacent to the repetitive sequence and a sequencing primer with 10 bp distance to the exon start is used according to the present invention. With this combination it is possible to reliably analyse at least two bp before the exon start using the forward primer and the whole intronic sequence up to the repeat with the reverse primer.

Mutation classification: The results of DNA sequencing may comprise changes of nucleotides. From these changes together with general knowledge of protein expression (mechanisms of translation), the impact on the function of the protein can be deduced. A base exchange at a certain position may create a stop codon, lead to the usage of another amino acid at this position or produce no apparent alteration. All these changes happen at the level of translation, but this base exchange may be already effective in mRNA processing. A translationally silent mutation may produce or disrupt a splice site as well as a binding site for regulatory factors (proteins, microRNAs). Furthermore very little is known about the relevance of intronic variants. Concerning splice site alterations a number of calculation programs are available to support the detection of a newly created or disrupted site (e.g.: Reese et al., J. Comp. Biol. 4 (1997), 311-323; Heebsgard et al., NAR 24 (1996), 3439-3452)). However, as the mechanism of splicing has not been resolved completely, none of these programs reflect the complete range of possible effects in vivo. It is widely accepted that a change within two base-pairs from the intron-exon-border impairs splicing and it is presumed to be also valid for the region of five base-pairs in each direction.

Based on these arguments all mutations qualify as functionally relevant in some way, unless they are known polymorphisms. Polymorphisms are present at a certain frequency in a population and have no (within an average lifespan of man not obvious) functional impact. Currently 85 polymorphisms in the p53 gene are listed in the IARC p53 database (http://www-p53.iarc.fr/PolymorphismView.asp).

Chemicals and kits used: PCR chemicals and enzymes are not restricted to those used in the present examples, but reaction conditions have to be optimised to obtain pure amplification products free of side products.

Quality control of PCR amplification can be done with any manual or automatic electrophoresis system available. Clean-up of PCR and sequencing products can be done using other methods and kits if quality of the result is provided. Sequence reaction and analysis can be transferred to systems from other supplier (e.g. Beckmann-Coulter CEQ) if the signal to noise ratio is adequate to detect variants in samples with a relatively high amount of normal DNA.

### II. Clinical evaluation using the method according to the present invention

Reports from exploratory studies regularly suggest potentially useful candidate markers to optimise and individualise cancer therapy. However, few markers are currently developed to the point of allowing reliable use in clinical practice. The lack of a disciplined approach will slow the introduction of markers into clinical use, or alternatively, markers may be introduced without sufficient scientific evidence of benefit. Once the marker meets the criterion of "promising", additional data must be gathered before initiating confirmatory studies to test its clinical utility. These data include the specificity of the marker to the cancer of interest (as opposed to normal tissues, other disease states, or other cancers), an estimate of the marker prevalence in the target population, confidence in the method of measurement, including definition of any cut points, and demonstration that the measurement can be reliably performed on the specimens that are available (Sargent et al., J. Clin. Oncol.23 (2005), 2020-2027).

Considering these development milestones a number of studies were performed to evaluate the clinical utility of p53 stepwise. While performing these studies (Examples II. 1-3) the qualitative interaction was detected and the two pathway model was developed. Both hypotheses are now finally tested in the first prospective randomised clinical phase III trial (pancho trial) appropriately designed to test for qualitative interaction.

### 1. The CRCLM (Colorectal cancer liver metastases) Study

Goal:
- Test for an independent association of the marker p53 and chemotherapy response
- get information about the prognostic properties of the marker by including an untreated control group
- get evidence for the qualitative interaction

Data on 76 patients with colorectal cancer liver metastases (CRCLM) were prospectively collected at a single institution between 2001 and 2003. Patients considered to be technically operable were included. Fifty-one patients received preoperative therapy with Oxaliplatin plus 5-FU and twenty-five were treated with surgery only. The groups did not differ in age, chronicity of CRCLM, staging and grading of the primary colorectal cancer. Treatment decision was based on the preference of the surgeon or the patient.

The p53 gene was assessed in all tumours through complete direct gene sequencing (exons 2-11 including splice sites) with the method according to the present invention.

In Figure 6A survival rates for the whole patient cohort are shown (the graph includes all patients with and without preoperative chemotherapy and separates them for harbouring p53 mutant and p53 normal tumours). In this graph a normal p53 seems to be beneficial. However, subgroup evaluation shows: Improved survival did occur in patients with p53 normal tumours but exclusively in the group receiving preoperative chemotherapy (p=0,025) (Figure 6B). The chemotherapy used was 5FU/Oxaliplatin. Both drugs belong to pathway 1 and need a normal p53 for induction of apoptosis.

In contrast, in the preoperatively untreated control group (Figure 6C), a normal p53 status in the tumour was not related to improved survival (p=0.543). Quite contrary, the effect seems to be inverse.

Comparing only the dotted lines of both subsets (Figures 6B and 6C), representing the patients with a p53 mutant tumour, patients with preoperative chemotherapy (Figure 6B) did much worse. These patients received drugs (5FU/Oxaliplatin) which belong to pathway 1. The graph shows that the pathway 1 chemotherapy was ineffective in patients with p53 mutated tumours. The graph additionally shows that pathway 1 chemotherapy harmed patients with p53 mutated tumours because patients with p53 mutated tumours and without preoperative chemotherapy did better.

Comparing the full lines of the two subsets with and without preoperative chemotherapy (representing the patients with p53 normal tumours), survival benefit was related to preoperative chemotherapy.

In summary, the used chemotherapeutic drugs belonging to pathway 1 interact positively with a normal p53 gene and negatively with a mutant p53 gene.

In other words, depending on the genotype of the marker (mutant or normal) the effect of therapy changes direction, which demonstrates the presence of a qualitative interaction. The degree of interaction is even stronger (p=0.0042) when the known prognostic parameters are considered in the statistical calculation (multivariate analysis). The p53 genotype shows a significant (qualitative) interaction with survival (response to therapy) in the chemotherapy treated subset only.

The p53 genotype of the tumour was only related to survival in patients receiving chemotherapy demonstrating that the marker p53 interacts with chemotherapy. This qualifies p53 as a predictive marker. P53 did not influence survival in the preoperatively untreated patients and therefore p53 does not qualify as a prognostic marker.

In summary, these results show that:
- The method according to the present invention is a reliable method for determination of the p53 status of a patient's tumour.
- p53 exclusively interacts with chemotherapy.
- p53 is not a prognostic marker but a marker predicting response to chemotherapy.
- p53 can easily be misinterpreted as a prognostic marker when the strong interaction with chemotherapy is not considered (see Fig 6A). Today almost all cancer patients are treated (pre or postoperatively) and most of the frequently used chemotherapies interact negatively with a mutant p53. Therefore in meta-analyses a mutant p53 status may appear as a bad prognostic marker.
- Any survival benefit attributed to p53 is based on its interaction with therapy.

### 2. The Oesophageal cancer PILOT STUDY

Goal:
- Validation trial for the relationship between marker genotype and outcome (finished 2007)
- test p53 adapted preoperative therapy (treatment considering the two pathway model) for the first time prospectively
- assess the true incidence of p53 mutations in oesophageal cancer with the method according to the present invention.

Background: Treatment of oesophageal cancer remains unsatisfactory. Cure rates are disappointingly low. The median survival time ranges around 17 months with a 3 ys survival rate of only 16%. Neither pre nor postoperative radio/chemotherapy in any combination proved to substantially improve this situation.

Only a small subgroup of patients who experience major response to preoperative therapy consistently shows a significantly increased survival. Using standard platinum-based regimen, yet about 15 % of patients can achieve pathological complete remission which translates in reported 3-year survival rates of 64 % in this group. Factors identifying this subgroup of responders and selecting optimal drugs for non-responders could therefore dramatically enhance treatment efficacy.

Methods: In order to test the hypothesis that the p53 genotype is predictive for chemotherapy response, a prospective study was conducted. Thirty-eight patients with potentially resectable esophageal cancer were evaluated for the relation between p53 genotype and response to two different neoadjuvant treatments. P53 gene mutations were assessed by complete direct sequencing of DNA extracted from diagnostic biopsies with the method according to the present invention. Response to neoadjuvant chemotherapy was assessed pathohistologically in the surgical specimen.

Results: 23 squamous cell carcinoma and 24 adenocarcinoma were included. 39 patients received standard therapy with CIS/5FU (Cisplatin 80mg/m2 d1 5-FU 1000mg/m2 d 1-5, q21,2 cycles). Eight patients received Docetaxel (75mg/m2, q21,2 cycles).

Presence of a p53 mutation was significantly associated with decreased survival in the group receiving 5FU/CIS and an increased survival in the group receiving Docetaxel (Fig 5B). Patients with a normal p53 gene experience a significant survival benefit after 5FU/CIS therapy.

| | CISPLATIN/5-FU | | | | | DOCETAXEL | | | |
|---|---|---|---|---|---|---|---|---|---|
| | | | | | | | | | |

| | P53 NORMAL | | P53 MUTANT | **P VALUE** | | P53 NORMAL | | P53 MUTANT | **P VALUE** |
|---|---|---|---|---|---|---|---|---|---|
| RESPONSE: CR, PR | **12** | | **0** | | | **0** | | **6** | |
| FAILURE: SD, PD | **2** | | **16** | <0.001 | | **2** | | **0** | 0.002 |

The overall response to p53 adapted neoadjuvant therapy was 94%. p53 adapted treatment was associated with a significant survival advantage (p=0,042) after a median follow up of 15.4 months (Fig 5E). There was no difference according to the different histological subtypes concerning the p53 interaction (Fig 5C, 5D).

Conclusion: These results are in concordance with our interaction and pathway model: As CIS/5FU belongs to pathway 1 and worked well in patients with a normal p53 gene in the tumour. Docetaxel belongs to pathway 2 and worked well in patients with p53 mutant tumours.

As a consequence, a prospective randomised trial - the PANCHO trial - was initiated to finally prove the interaction between the predictive marker p53 and response to CIS/5-FU and Docetaxel, respectively.

### 3. The PANCHO Trial

Goal:
- Provide clinical evidence for the qualitative interaction and the two pathway model.
- prove the clinical utility of p53 for the first time in a prospective randomised, clinical phase III trial.
- use the Marker by treatment interaction design proposed by Sargent and test for interaction between p53 and response to therapy for the first time in the context of a phase III trial

PANCHO = "p53 adapted neoadjuvant chemotherapy for operable oesophageal cancer" EudraCT 2006 006647-31, NCT00525200) is an ongoing clinical, predictive marker trial conducted by the p53 research group (started 2007, scheduled to be finished 2011).

The trial was designed to provide clinical evidence for the two pathway model and the qualitative interaction of p53 and anticancer therapy.

There is no single marker known for which a direct qualitative interaction with cancer therapy has ever been shown in a clinical trial. Additionally, based on the two pathway model of p53 interacting with cancer therapy, this interaction is two sided.

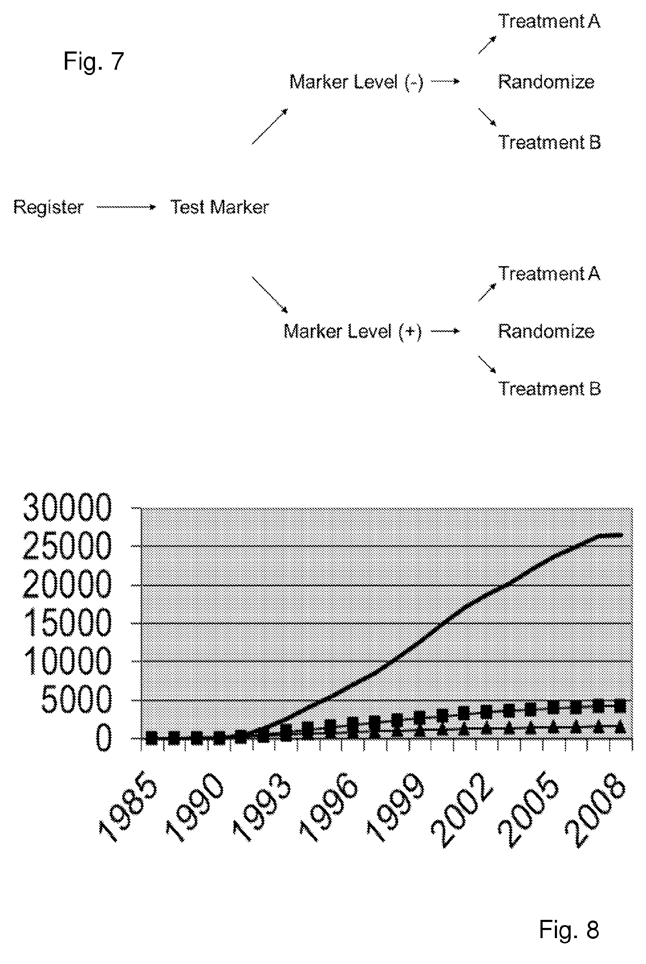

The design of the pancho trial - marker by treatment interaction design (Sargent et al., J. Clin. Oncol. 23 (2005), 2020-2027) - was proposed as the statistically adequate design to test a possible interaction between a marker and response (Fig. 7 and 9).

Until the present invention this design has never been used in a clinical trial because still no marker has been identified to meet the prerequisites: availability of a potential effective therapy for each of the two marker expressions (mutant or wild type) which generates a huge difference in response. The qualitative two sided interaction model according to the present invention will change the standards of cancer therapy, reducing toxicity while improving efficacy.

## Claims

1. Method for determining the p53 status of a tumour patient **characterised in** by the following steps:
- detecting in a sample of body fluid or a tissue sample of the tumour patient containing tumour cells or cell-free tumour DNA; said tumour cells or cell-free tumour DNA containing DNA molecules whether the p53 gene is present in native form on said DNA molecules or whether the p53 gene on said DNA molecules has one or more mutations; said detecting being carried out by:
- performing on the DNA from said tumour cells or cell-free tumour DNA a quality-controlled, triplicate multiplex polymerase chain reaction (PCR) covering the p53 gene of Seq.ID No. 1 in the region from bp 11619 to bp 18741, especially in the region from bp 11689 to bp 18680, thereby generating multiplex PCR amplification products;
- determining the sequence of said triplicate multiplex PCR amplification products by using forward and reverse primers for sequencing thereby generating the sequence of the p53 gene in this region of said tumour cells or cell-free tumour DNA; and
- comparing the generated sequence with a native p53 gene sequence to detect whether there is at least one mutation present in said tumour cells or cell-free tumour DNA; and
- determining the p53 status of said tumour patient as mutated or native, depending on whether at least one mutation was detected in the nucleic acids of said tumour cells or cell-free tumour DNA,
wherein all primer pairs according to SEQ.ID.Nos. 2 and 4 to 24 are used in said triplicate multiplex PCR and/or said sequence determination.

2. Method according to claim 1, **characterised in that** said multiplex PCR is performed with primers having a melting temperature of 58°C to 72°C, preferably of 60°C to 70°C, especially of 65°C to 68°C.

3. Method according to claim 1 or 2, **characterised in that** said multiplex PCR is performed with at least 10, preferably at least 15, especially at least 20, primer pairs covering different regions of the p53 gene.

4. Method according to any one of claims 1 to 3, **characterised in that** said multiplex PCR is performed with 5 or less independent PCRs, preferably with 4 or less independent PCRs, especially with 3 or less independent PCRs.

5. Method according to any one of claims 1 to 4, **characterised in that** a positive and a negative control is run in parallel to the determination of the p53 status of the tumour patient.

6. Method according to any one of claims 1 to 5, **characterised in that** a tumour cell or a cell-free DNA with a p53 gene in native form and/or a tumour cell or a cell-free DNA with a mutated p53 gene is used as a positive control.

7. Method according to any one of claims 1 to 6, **characterised in that** a negative control is run in parallel to the determination of the p53 status of the tumour patient and said negative control is DNA free of sequences that are amplified during the triplicate multiplex PCR and/or a DNA free solution.

8. Method according to any one of claims 1 to 7, **characterised in that** for the triplicate multiplex PCR and for the determination of the sequence of said triplicate multiplex PCR amplification products the same primers are used, preferably with the exception of exon 4.

9. Kit for performing the method according to any one of claims 1 to 8, **characterised in that** it comprises:
- a PCR primer set;
- optionally PCR reagents, including a DNA Polymerase, buffer(s), and dNTPs; and
- a sequencing primer set, said kit containing the primers with SEQ.ID.Nos. 2 to 24.

10. Kit according to claim 9, **characterised in that** it further comprises control reagents, preferably positive control reagents, especially a tumour cell or a cell-free DNA with a p53 gene in native form and/or a tumour cell or a cell-free DNA with a mutated p53 gene, or negative control reagents, DNA free of sequences that are amplified during the triplicate multiplex PCR and/or a DNA free solution.

11. Kit according to claim 9 or 10, **characterised in that** it comprises a PCR thermocycler.

12. Kit according to any one of claims 9 to 11, **characterised in that** it comprises prepared multiplex mixtures of primers.

## Patentansprüche

1. Verfahren zum Bestimmen des p53-Status eines Tumorpatienten, **gekennzeichnet durch** die folgenden Schritte:
- das in einer Probe von Körperflüssigkeit oder Gewebe des Tumorpatienten, die Tumorzellen oder zellfreie Tumor-DNA enthält, wobei die Tumorzellen bzw. die zellfreie Tumor-DNA DNA-Moleküle enthalten, erfolgende Detektieren, ob das p53-Gen in nativer Form auf den DNA-Molekülen vorliegt bzw. ob das auf den DNA-Molekülen vorliegende p53-Gen eine oder mehrere Mutationen aufweist; wobei das Detektieren erfolgt **durch**:
- das Durchführen einer qualitätskontrollierten, Dreifach-Multiplex-Polymerase-Kettenreaktion (PCR) an der DNA aus den Tumorzellen bzw. der zellfreien Tumor-DNA, wobei das p53-Gen gemäß SEQ ID NR: 1 in der Region von bp 11619 bis bp 18741, insbesondere in der Region von bp 11689 bis bp 18680 abgedeckt wird, wodurch Multiplex-PCR-Amplifikationsprodukte erzeugt werden;
- das Bestimmen der Sequenz der Dreifach-Multiplex-PCR-Amplifikationsprodukte unter Verwendung von Vorwärts- und Rückwärtsprimern zur Sequenzierung, wodurch die Sequenz des p53-Gens in dieser Region der Tumorzellen bzw. der zellfreien Tumor-DNA erzeugt wird; und
- das Vergleichen der so erzeugten Sequenz mit einer nativen p53-Gensequenz, um zu detektieren, ob in den Tumorzellen bzw. der zellfreien Tumor-DNA mindestens eine Mutation vorliegt; und
- das Bestimmen des p53-Status des Tumorpatienten als mutiert oder nativ in Abhängigkeit davon, ob mindestens eine Mutation in den Nukleinsäuren der Tumorzellen bzw. der zellfreien Tumor-DNA detektiert wurde,
wobei bei der Dreifach-Multiplex-PCR und/oder der Sequenzbestimmung alle Primerpaare gemäß den SEQ ID NRs: 2 und 4 bis 24 verwendet werden.

2. Verfahren nach Anspruch 1, **dadurch gekennzeichnet, dass** die Multiplex-PCR mit Primern durchgeführt wird, die über eine Schmelztemperatur von 58°C bis 72°C, bevorzugt von 60°C bis 70°C, insbesondere von 65°C bis 68°C verfügen.

3. Verfahren nach Anspruch 1 oder 2, **dadurch gekennzeichnet, dass** die Multiplex-PCR mit mindestens 10, bevorzugt mit mindestens 15, insbesondere mit mindestens 20 Primerpaaren durchgeführt wird, die jeweils unterschiedliche Regionen des p53-Gens abdecken.

4. Verfahren nach einem der Ansprüche 1 bis 3, **dadurch gekennzeichnet, dass** die Multiplex-PCR mit 5 oder weniger voneinander unabhängigen PCRs, bevorzugt mit 4 oder weniger voneinander unabhängigen PCRs, insbesondere mit 3 oder weniger voneinander unabhängigen PCRs durchgeführt wird.

5. Verfahren nach einem der Ansprüche 1 bis 4, **dadurch gekennzeichnet, dass** parallel zu der Bestimmung des p53-Status des Tumorpatienten eine positive und eine negative Kontrolle durchgeführt werden.

6. Verfahren nach einem der Ansprüche 1 bis 5, **dadurch gekennzeichnet, dass** eine Tumorzelle oder eine zellfreie DNA mit einem p53-Gen in nativer Form und/oder eine Tumorzelle oder eine zellfreie DNA mit einem mutierten p53-Gen als positive Kontrolle verwendet werden.

7. Verfahren nach einem der Ansprüche 1 bis 6, **dadurch gekennzeichnet, dass** parallel zu der Bestimmung des p53-Status des Tumorpatienten eine negative Kontrolle durchgeführt wird und es sich bei der negativen Kontrolle um DNA, die frei von Sequenzen ist, die im Verlauf der Dreifach-Multiplex-PCR amplifiziert werden, und/oder um eine DNA-freie Lösung handelt.

8. Verfahren nach einem der Ansprüche 1 bis 7, **dadurch gekennzeichnet, dass** sowohl für die Dreifacch-Multiplex-PCR als auch für die Bestimmung der Sequenz der Dreifach-Multiplex-PCR-Amplifikationsprodukte jeweils die gleichen Primer verwendet werden, bevorzugt mit der Ausnahme von Exon 4.

9. Kit zur Durchführung des Verfahrens nach einem der Ansprüche 1 bis 8, **dadurch gekennzeichnet, dass** das Kit umfasst:
- einen Satz von PCR-Primern;
- gegebenenfalls PCR-Reagenzien, einschließend eine DNA-Polymerase, einen oder mehrere Puffer sowie dNTPs; und
- einen Satz von Sequenzierungsprimern, wobei das Kit die Primer mit den SEQ ID NRs: 2 bis 24 enthält.

10. Kit nach Anspruch 9, **dadurch gekennzeichnet, dass** das Kit des Weiteren Kontrollreagenzien umfasst, und zwar bevorzugt positive Kontrollreagenzien, insbesondere eine Tumorzelle oder eine zellfreie DNA mit einem p53-Gen in nativer Form und/oder eine Tumorzelle oder eine zellfreie DNA mit einem mutierten p53-Gen, oder negative Kontrollreagenzien, DNA, die frei von Sequenzen ist, die im Verlauf der Dreifach-Multiplex-PCR amplifiziert werden, und/oder eine DNA-freie Lösung.

11. Kit nach Anspruch 9 oder 10, **dadurch gekennzeichnet, dass** das Kit einen PCR-Thermocycler umfasst.

12. Kit nach einem der Ansprüche 9 bis 11, **dadurch gekennzeichnet, dass** das Kit vorgefertigte Multiplex-Primergemische umfasst.

## Revendications

1. Procédé de détermination du statut de p53 d'un patient présentant une tumeur **caractérisé par** les étapes suivantes :
- le fait de détecter, dans un échantillon de fluide corporel ou un échantillon de tissu du patient présentant une tumeur contenant des cellules tumorales ou de l'ADN tumoral exempt de cellules, lesdites cellules tumorales ou ledit ADN tumoral exempt de cellules contenant des molécules d'ADN, si le gène p53 est présent sous une forme native sur lesdites molécules d'ADN ou si le gène p53 sur lesdites molécules d'ADN comporte une ou plusieurs mutations ; ladite détection étant réalisée par :
- la réalisation, sur l'ADN desdites cellules tumorales ou l'ADN tumoral exempt de cellules, d'une réaction en chaîne par polymérase (PCR) multiplexe en triple, avec un contrôle qualité, couvrant le gène p53 de SEQ ID NO: 1 dans la région comprise entre la pb 11619 et la pb 18741, particulièrement dans la région comprise entre la pb 11689 et la pb 18680, en générant ainsi des produits d'amplification de PCR multiplexe ;
- la détermination de la séquence desdits produits d'amplification de PCR multiplexe en triple en utilisant des amorces sens et antisens pour un séquençage, en générant ainsi la séquence du gène p53 dans cette région desdites cellules tumorales ou dudit ADN tumoral exempt de cellules ; et
- la comparaison de la séquence générée avec une séquence du gène p53 natif afin de détecter si au moins une mutation est présente dans lesdites cellules tumorales ou ledit ADN tumoral exempt de cellules ; et
- la détermination du statut de p53 dudit patient présentant une tumeur comme étant muté ou natif, selon si au moins une mutation a été détectée dans les acides nucléiques desdites cellules tumorales ou dudit ADN tumoral exempt de cellules,
où toutes les paires d'amorces selon SEQ ID NO: 2 et 4 à 24 sont utilisées dans ladite PCR multiplexe en triple et/ou ladite détermination de séquence.

2. Procédé selon la revendication 1, **caractérisé en ce que** ladite PCR multiplexe est réalisée avec des amorces ayant une température de fusion de 58 °C à 72 °C, de préférence de 60 °C à 70 °C, particulièrement de 65 °C à 68 °C.

3. Procédé selon la revendication 1 ou 2, **caractérisé en ce que** ladite PCR multiplexe est réalisée avec au moins 10, de préférence au moins 15, particulièrement au moins 20, paires d'amorces couvrant différentes régions du gène p53.

4. Procédé selon l'une quelconque des revendications 1 à 3, **caractérisé en ce que** ladite PCR multiplexe est réalisée avec 5 PCR indépendantes ou moins, de préférence avec 4 PCR indépendantes ou moins, particulièrement avec 3 PCR indépendantes ou moins.

5. Procédé selon l'une quelconque des revendications 1 à 4, **caractérisé en ce qu'**un contrôle positif et négatif est analysé en parallèle à la détermination du statut de p53 du patient présentant une tumeur.

6. Procédé selon l'une quelconque des revendications 1 à 5, **caractérisé en ce qu'**une cellule tumorale ou un ADN exempt de cellules avec un gène p53 sous une forme native et/ou une cellule tumorale ou un ADN exempt de cellules avec un gène p53 muté est utilisé(e) en tant que contrôle positif.

7. Procédé selon l'une quelconque des revendications 1 à 6, **caractérisé en ce qu'**un contrôle négatif est analysé en parallèle à la détermination du statut de p53 du patient présentant une tumeur et ledit contrôle négatif est de l'ADN exempt de séquences qui sont amplifiées pendant la PCR multiplexe en triple et/ou une solution exempte d'ADN.

8. Procédé selon l'une quelconque des revendications 1 à 7, **caractérisé en ce que** pour la PCR multiplexe en triple et pour la détermination de la séquence desdits produits d'amplification de la PCR multiplexe en triple, les mêmes amorces sont utilisées, de préférence à l'exception de l'exon 4.

9. Kit pour mettre en oeuvre le procédé selon l'une quelconque des revendications 1 à 8, **caractérisé en ce qu'**il comprend :
- un jeu d'amorces de PCR ;
- facultativement des réactifs de PCR, comprenant une ADN polymérase, un/des tampon(s), et des dNTP ; et
- un jeu d'amorces de séquençage, ledit kit contenant les amorces de SEQ ID NO: 2 à 24.

10. Kit selon la revendication 9, **caractérisé en ce qu'**il comprend en outre des réactifs de contrôle, de préférence des réactifs de contrôle positif, particulièrement une cellule tumorale ou un ADN exempt de cellules avec un gène p53 sous une forme native et/ou une cellule tumorale ou un ADN exempt de cellules avec un gène p53 muté, ou des réactifs de contrôle négatif, de l'ADN exempt de séquences qui sont amplifiées pendant la PCR multiplexe en triple et/ou une solution exempte d'ADN.

11. Kit selon la revendication 9 ou 10, **caractérisé en ce qu'**il comprend un thermocycleur de PCR.

12. Kit selon l'une quelconque des revendications 9 à 11, **caractérisé en ce qu'**il comprend des mélanges d'amorces multiplexes préparés.


### SEQUENCE LISTING

<110> Kandioler, Daniela Prof.
<120> Response Prediction in Cancer Treatment
<130> R 55959
<160> 24
<170> PatentIn version 3.5
<210> 1
   <211> 20303
   <212> DNA
   <213> Homo sapiens
<400> 1
   ttcccatcaa gccctagggc tcctcgtggc tgctgggagt tgtagtctga acgcttctat      60
   cttggcgaga agcgcctacg ctccccctac cgagtcccgc ggtaattctt aaagcacctg     120
   caccgccccc ccgccgcctg cagagggcgc agcaggtctt gcacctcttc tgcatctcat     180
   tctccaggct tcagacctgt ctccctcatt caaaaaatat ttattatcga gctcttactt     240
   gctacccagc actgatatag gcactcagga atacaacaat gaataagata gtagaaaaat     300
   tctatatcct cataaggctt acgtttccat gtactgaaag caatgaacaa ataaatctta     360
   tcagagtgat aagggttgtg aaggagatta aataagatgg tgtgatataa agtatctggg     420
   agaaaacgtt agggtgtgat attacggaaa gccttcctaa aaaatgacat tttaactgat     480
   gagaagaaag gatccagctg agagcaaacg caaaagcttt cttccttcca cccttcatat     540
   ttgacacaat gcaggattcc tccaaaatga tttccaccaa ttctgccctc acagctctgg     600
   cttgcagaat tttccacccc aaaatgttag tatctacggc accaggtcgg cgagaatcct     660
   gactctgcac cctcctcccc aactccattt cctttgcttc ctccggcagg cggattactt     720
   gcccttactt gtcatggcga ctgtccagct ttgtgccagg agcctcgcag gggttgatgg     780
   gattggggtt ttcccctccc atgtgctcaa gactggcgct aaaagttttg agcttctcaa     840
   aagtctagag ccaccgtcca gggagcaggt agctgctggg ctccggggac actttgcgtt     900
   cgggctggga gcgtgctttc cacgacggtg acacgcttcc ctggattggg taagctcctg     960
   actgaacttg atgagtcctc tctgagtcac gggctctcgg ctccgtgtat tttcagctcg    1020
   ggaaaatcgc tggggctggg ggtggggcag tggggactta gcgagtttgg gggtgagtgg    1080
   gatggaagct tggctagagg gatcatcata ggagttgcat tgttgggaga cctgggtgta    1140
   gatgatgggg atgttaggac catccgaact caaagttgaa cgcctaggca gaggagtgga    1200
   gctttgggga accttgagcc ggcctaaagc gtacttcttt gcacatccac ccggtgctgg    1260
   gcgtagggaa tccctgaaat aaaagatgca caaagcattg aggtctgaga cttttggatc    1320
   tcgaaacatt gagaactcat agctgtatat tttagagccc atggcatcct agtgaaaact    1380
   ggggctccat tccgaaatga tcatttgggg gtgatccggg gagcccaagc tgctaaggtc    1440
   ccacaacttc cggacctttg tccttcctgg agcgatcttt ccaggcagcc cccggctccg    1500
   ctagatggag aaaatccaat tgaaggctgt cagtcgtgga agtgagaagt gctaaaccag    1560
   gggtttgccc gccaggccga ggaggaccgt cgcaatctga gaggcccggc agccctgtta     1620
   ttgtttggct ccacatttac atttctgcct cttgcagcag catttccggt ttctttttgc     1680
   cggagcagct cactattcac ccgatgagag gggaggagag agagagaaaa tgtcctttag     1740
   gccggttcct cttacttggc agagggaggc tgctattctc cgcctgcatt tctttttctg     1800
   gattacttag ttatggcctt tgcaaaggca ggggtatttg ttttgatgca aacctcaatc     1860
   cctccccttc tttgaatggt gtgccccacc ccccgggtcg cctgcaacct aggcggacgc     1920
   taccatggcg tagacaggga gggaaagaag tgtgcagaag gcaagcccgg aggcactttc     1980
   aagaatgagc atatctcatc ttcccggaga aaaaaaaaaa agaatggtac gtctgagaat     2040
   gaaattttga aagagtgcaa tgatgggtcg tttgataatt tgtcgggaaa aacaatctac     2100
   ctgttatcta gctttgggct aggccattcc agttccagac gcaggctgaa cgtcgtgaag     2160
   cggaaggggc gggcccgcag gcgtccgtgt ggtcctccgt gcagccctcg gcccgagccg     2220
   gttcttcctg gtaggaggcg gaactcgaat tcatttctcc cgctgcccca tctcttagct     2280
   cgcggttgtt tcattccgca gtttcttccc atgcacctgc cgcgtaccgg ccactttgtg     2340
   ccgtacttac gtcatctttt tcctaaatcg aggtggcatt tacacacagc gccagtgcac     2400
   acagcaagtg cacaggaaga tgagttttgg cccctaaccg ctccgtgatg cctaccaagt     2460
   cacagaccct tttcatcgtc ccagaaacgt ttcatcacgt ctcttcccag tcgattcccg     2520
   accccacctt tattttgatc tccataacca ttttgcctgt tggagaactt catatagaat     2580
   ggaatcagga tgggcgctgt ggctcacgcc tgcactttgg ctcacgcctg cactttggga     2640
   ggccgaggcg ggcggattac ttgaggatag gagttccaga ccagcgtggc caacgtggtg     2700
   aatccccgtc tctactaaaa aatacaaaaa ttagctgggc gtggtgggtg cctgtaatcc     2760
   cagctattcg ggagggtgag gcaggagaat cgcttgaacc cgggaggcag aggttgcagt     2820
   gagccaagat cgtgccacta cactccagcc tgggcgacaa gaacgaaact ccgtctcaaa     2880
   aaaaaggggg gaatcataca ttatgtgctc atttttgtcg ggcttctgtc cttcaatgta     2940
   ctgtctgaca ttcgttcatg ttgtatatat cagtattttg ctccttttca tttagtatag     3000
   tccatcgatt gtatatccgt ccttttgatg gccttttgag ttgtttccca tttgcggtta     3060
   tgaaataaag ctgctataaa cattcttgta caattctttt tgtgatcata tgttttcgtg     3120
   tttcttggag aaatacttag gaggggaatt gtggaggaag taaaaagtag ctgtattttg     3180
   aactttttca gaagctctga gttttccaga gcggttgtac cattttacac tccaactagc     3240
   aaggtatggg agttattatg gttgtgccac agccttccgg acattaggta tgtcagtctt     3300
   tctaatgtgg tatatccttg tggttgtaat ttacagttct ctattgacta aggatgttca     3360
   gcattttttc atgtgcctat tggccattcg tattttgttt gtaaagtagc tcttcgagtc     3420
   ttttacctgt tattttggtt tttttgtttg tttttattgt tcagttgtgg gactgcttta     3480
   tactttctgg atacaagtcc ttatcagatc catgagtcgt gaatgttttc ttctgatctg     3540
   ttgcgggcct atttgtttgc tttacagagt ttacagaatc ttaagaggag tggattaatc     3600
   ttttttatgt tcagtatttg ccttgtcctg tttaggacat cttttttttt ttttttaacc     3660
   ccagggtcat gaagatataa tcttacattt tcttttagga cctttatggt ggtaagtttt 3720
   acagtaaggt ccttaagcca ttaattaatt cttaaaatta attgtttatg gtgtgaggtg 3780
   taggagtcag tctctggtat ctttcctgta tggaaatcca gttattctgt ctccacttgt 3840
   tgaaataggc ttcctttctc tactgaatgc ttttaatttt aattatttta cagttggagt 3900
   atagggctac cattttagtg ctattttctt tttttctttg ttaatttttg agacagggac 3960
   tcacactgtt gcccaggcta gagtacaatg gcacaatcaa ggcttactgc agcctcgaac 4020
   ccctgggctc aagcagtcct ctagcagcct cacgagtagc tgggattact ccaccacacc 4080
   cagctaacta ttttattttt ttgtattgac aggatctcac tatgttgccc aggctggtct 4140
   caaactgctg gcctcaagct ttcatcccat ctcggcctcc caaagtgctg ggattacagg 4200
   tgtgagccac catgcctgac ctcttagtgc tattttctat ttatctcctc tgttctctgc 4260
   tctctttaaa cgttggagga agaaacagta cccatcttac acaaactctt cagaaaacag 4320
   aggaacagac tgggcgcggt ggctcatacc tgtaatctca gcactttggt acgctgaggc 4380
   aggggatcat ttgaggtcgg gagttcgaga ccagcctggc caacacggcg aaaccccatc 4440
   tctactaaaa tacaaaagta gctaggcgtg caccatacct gtaatgccag ttactcagga 4500
   ggctgaggca caagaatccc ttgaacctgg gaagcggagg ttgcagtgag ccgagattgc 4560
   gccactgcac tccagcctgg gcaacagagt gagaccctgt ctcagaaaaa aaaagaaaga 4620
   aagaaaaaat agaggaatat ttcccaactt gttttcgaag ccaggataat cctggtacca 4680
   aaaccaaaca aggacattat aagaaaagaa aatatagacc aatattcctg ttagcataga 4740
   catgcaacag ctaaccaatt ttagcaaacc aaacctggta atatagaaaa aaggataaat 4800
   aggccagtcg cggtggctca cgcctgtaat cccagcactt tgggaggctg aggcaggcag 4860
   atcacttgag gtcaggagtt tgagaccagc ctgaccaaca tggtgaaacc ccgtttctaa 4920
   taaaaataca aaaatcaggc tgggcacggt ggctcacgcc tgtaatccca gcactttggg 4980
   aggccgaggt gggcagatca cgaggtcagg agttcaagac cagcctgacc aatgtggtga 5040
   aacgccatct ctactaaaaa tacgaaaatc agccggtgtg gtggcacctg cctgtaatcc 5100
   cagctactca ggaggctgag gcagaattgc ttgaacccgg gaggcagagg ttgcagtgag 5160
   ccaagatcgt gccactgcac tccagcctgg gcgacagagc aagacttcat ctcaaaaaaa 5220
   aaaaaaatta gctgggcatg gtggtgggca cctgaaatcc cagctactcg ggagtctgag 5280
   gcaggagaat cgcttgaacc caggaggcag aagttgcact gagctgggat cacaccattg 5340
   cactccagcc tgggcaacag agtgagactc catctcaaaa aaagaaaaag aaaaaggata 5400
   aatacattct aaccaaataa tgtttatctc atgattgtag ctgattcaac attcaaaaat 5460
   tggcctggtg cagtagctca ggcctgtaat cccaacattt taggaggctg aggcaggaag 5520
   atctcttgag cccaggattt caagaccagc ctgggcaaca tagtcagact ggtctttact 5580
   ggggggaaaa aaatcagtct gtgtaattca ccacattaac aaagggaaac ataaaaaccc 5640
   tatgatcatt tcaacagatg tagcaaaagc agttaatgat atcaacacat atgcatgatt 5700
   acaaaccaac caacctccta gcaaactagg gaaaggaaac ttaactagtt tgataacagg 5760
   gcgtccacag tcggagttcc actagcagca tacataatgg tagaaaactc agtgctgctg 5820
   ggggcggtgg ctcacgcctg taatgccagc gctttgggag gcctaggcgg gcggatcacg 5880
   aggtcaggag atcgagactg tcctgactag catgctgaaa ccccgtctct actaaaaata 5940
   caaaaacaaa aaattagccg ggcatggtgg cgggcgccta tagtgccagc tactcgggag 6000
   gctgaggcga gagaatggcg tgaacccggg aggcggagct tgcagagcct agatcgtgcc 6060
   actgcactcc agcctgggtg acagagtgag acttcgtctc aaaaaaaaaa aaaaaaaaaa 6120
   aagaaaagaa aactcaacgc tttttcctct aagatcagga actagaaaag gatttgactc 6180
   tcacaacgtt gataccatac tggaggtttt aaccaggcaa gaaaaagaaa taatgagggc 6240
   cgggtgcggt ggctcaggcc tgtaatccca gcactttggg aagccgagac gggtggatca 6300
   cgaggtcagg agatcgagcc atcctggcta acacggtgaa accctgtctc tactaaatat 6360
   acaaaaaatt agccgggcgt ggtggcgggc gcctgtagtc ccagctactc gggaggctga 6420
   ggcaggagaa tggcgtgaac tcagggggcg gagcttgcag tgagctgaga tcgagccact 6480
   gcactccagc ctgggcgaca gagcaagact gtgtctcaaa aaaaaaaaaa gaaaaagaaa 6540
   taatgattag tggcccgatg tctcacgcca gtaatcccag cactttggga ggccgaggtg 6600
   ggcagatcac ctgaggtctg gagttggaga ccagcctgac aaagatggtg aaacctcgtc 6660
   tctattaaaa tattaaaaaa atagccaggc gttggccggg tacagtggct catgcctgta 6720
   accccagcac tttgggaggc cgaggtgggt ggatcacctg aggtcaggag ttcaacacca 6780
   gcctggccaa catggtgaaa ccccatctct actaaaaata caaaattagc cgggcgtagt 6840
   ggcgggcgcc tgtaatccca gctacttggg aggcttaggc aggagaatcg cttgaacctg 6900
   ggaggcggag gttgtagtga gccgagattg caccattgca ctccagcctg ggtgacaaaa 6960
   gcaaaaactc cgtctcaaaa aaaaaagaat tagccagggg tagtggtgaa cgcctgtagt 7020
   cccagctact caggaggcag aggcaggaga atcacttgaa ccccggaggc agaggttgca 7080
   gtgagccgag attgtcccat tgcactccag cctaggcgag aagagcaaaa ttccatgtca 7140
   aaaaaaaaaa aaaaaaagga aagaaaaaaa ataacgatta gaaaggaaga aatcaaacac 7200
   attcacagcc agtatgattc tatacatacc atggtcctaa tggggccagg cgtggtggct 7260
   catgctgtaa tcctagcact tttaggaggc tgaggcaggt ggcttccctg ggaccagctg 7320
   gccaacatgg tgaaacccca actctaataa aaatacaaaa aatcagccag gcgtggtgag 7380
   ggcacctcta atcccagcta ctcaggaggc tgaggcagga gaattgcttg gacctgggag 7440
   gcagaggttg cagtgagccg agatcgcgct attgcactcc agcctgggca acaagagtga 7500
   aactccggca gggtgtggtc ttacgcctgt aatcccagca cttcgggagg ctgagccagg 7560
   ccgatcacct gaggtcagga gtttgagacc aacctaacat ggtgaaaccc cgtctctact 7620
   aaaaatacaa gaattagctg ggtgtagtgg tgggcgcctg taatcccagc tacttgggag 7680
   gctgagacag aagaattgct tgaacccagg aggtggaggt tgcagtgagc tgagatcatg 7740
   ccattgcaca ccacgccggg caacagagcg agattccgtc tcaaaaaaaa aaaaaagatg 7800
   aaactctatc tcaaaaaaaa aaaaaagtcc taatggaaaa tccataaaaa gctaccaaaa 7860
   ctaataaata aatatagcag ggttgcaggt tacagggcaa tatagttatc cctctatctg 7920
   taggggcttg gttctgggac tcctcacaca ccaaacccac agatgtctaa gtcccatata 7980
   taagacggaa tagtatttaa cctacacata tcctcccata tagtttaaat tatctagatt 8040
   acttacatta cccccataca atgaaaatgc taatgtacat gcaagtatgt atgtaagtac 8100
   ttgtactata ttgtttaggg aatcactgga cagataggcc ttcaagactg ataccagcag 8160
   ccactgttaa gattctggtc aggcctgccc ctgtttgggg tctcagttga tctcattgcc 8220
   ttcccaccca gccaagggca cctgcatttc tcttggctcc ctggccattt ggaaggccta 8280
   gttcagcctg gcacatttgt atcctggccc actgatgctg gtacccctgg gaaggtcctg 8340
   ctctgaaaaa cacggagatt ttagttgcta ctgaagattt gagagataaa gacagggaga 8400
   cctgtctgta gacctgtgtc cctccaagtg ggattgagac tttgggcccc ccatttcagg 8460
   acagcacctc ctggcctgtt gactgaatag atccctgaag gaggtgtagt tgcattttag 8520
   gagtgggggt gggagcagta ccactgatcc gcactaacaa tcacacagtt ctctctagaa 8580
   taataatata gaacaagtga aatagaacaa ttgcagaaag agctaacctt tgttgagctc 8640
   ttactgtgtg cccagcactt tcctcaactc tacatttccc ataatacata gagtactagg 8700
   taggcggggc ttgggggctc acgcctgtaa tcccagcact ttaggaggcc aaggggggtg 8760
   gatcacctga ggtcgggagt tcaagaccag cctgactaac atggtgaaac cccgtctcta 8820
   ctagaagtac aaaattagcc aggtgtggtg gcacatgctt gtagtcctag ctactcagca 8880
   ggctgaggca ggagaatcat ttgaatccgg gaggaggttg cagtaagcgg agatagtgcc 8940
   actgtactcc agcctgggca ataagagctg agactccgtc tcaaaataaa ataaaataaa 9000
   ataaaataaa ataaaataaa ataaaaaaag aaaagagcct gccattaaag gagctgtttg 9060
   gtaggggatg ttttgtcagt gcaaacaaca gaaaagtggg ctgggcacag tggttcatgc 9120
   ctgtaatccc agcactttgg gaggccaagg cgggcggatc acctgaagtt gggagttcaa 9180
   gaccagcctg accaatatgg agaaaccccg tctctactaa aaatacaaaa ttagccgggc 9240
   gcagtggccg atgcctgtaa tcccagctac tcgggaggct gaggcaggag aatcgcttga 9300
   acctgggagg cagaggttgc ggtgagccga gatcgcacca ttgcactcca gcctggacga 9360
   gagcaaaact ctgtctcaaa aaaaaaaaaa aacagaaaag tgtaacaaac acttacagta 9420
   ggcatgtttc ttagcaaatc tgatgacaaa tttggcataa agaaagagag catccctgaa 9480
   aaaaaaaaaa agaaaaagaa agagagcatc ctgcctgggc aacatagtga aaccctgcct 9540
   ctacaaaaaa actcaaaaat tggccgggtg cagtggctca cacctgtaat cccagcactt 9600
   tgggagtcgg aggcgggagg atcacctgag gtcaggagtt cgaaaccagc ctggccaaca 9660
   tggcaaaacc ccatctctac taaaaataca aaaaattaat caggcgcatt ggtgggcgcc 9720
   tgtaatccca gctactcagg aagttgaggc aagaggatcg cttgatactg ggaggtggag 9780
   gttacagtga gtcgagatca caccactgca ctctagcctg ggtgacaggg cgagactccg 9840
   tctccaaaaa aaaaaagaaa aagaaaaaga ctaaaaaatt agccaggcag gcctctgtgg 9900
   tcccagctac ttgggaggct gaggcaggag aatcactgag cccaggagtg gcaggctgta 9960
   gtgagccatg attgcaccac tgtaccctag cttgggcttc aaagcaagac cctgcctcaa 10020
   aagaaaaaag aaagaaagaa agaacatggc gggccaggca cagtggctca cacctgtaat 10080
   cccagcgctt tgagaggccg aggcaggtgg atcacaaggt caggagttcc acaccagcct 10140
   ggccaacatg gtgaaaccct gtctctacta aaaatacaaa aaatcagcag gcagggtggt 10200
   aggggcctgt aatcccagct actcgggagg ctgaggcagg agaattgctt gaaaccagaa 10260
   ggcagaggtt gcagtgagcc tagactgcac cactgcactc cagcctgggc gaaaagagcc 10320
   aaactccatc tcaaaaaaca aacaaaaaaa caaaacaaaa aaaaacatgg cagcctttga 10380
   aagcttgtct gggagaaggt gcgatgatgg ttgcataact tcgtgcaaga tgctggtcca 10440
   cacaggggct gccccttgct ctttctcgct ctcttaacct ctcatataac aggcttgtgt 10500
   gttatgcaca tttattgagc ccaagcaggt gcaaggcatt gtgatctaat actttggtca 10560
   gcaagacaac aagatagatc actgccctgc ccttaggaag tgtatatgct attagaggaa 10620
   acagataaaa taaacaagga aaagtatcag acaatgtaag tgctatgaga atgcaaatga 10680
   ggtgatgtga attaaaatag gatgacttaa gtctgcacgg aaggccccta cccccatgtt 10740
   cctggctagc caaggaacca ccagttgatt agcagagaag ggcagcccgt ctagctagag 10800
   cttttgggga agagggagtg gttgttaaga gatgagatta aagaagccga gacgggccct 10860
   tcgtgagggg gggttgtaat gcagggctga ggagtgtccg aagagaatgg gcaggtgagc 10920
   ggtgagacag ttgttcttcc agaagctttg cagtgaaagg aatcaaagaa atggagccgt 10980
   gtatcaggtg gggaagggtg ggggccaagg gggtgtcctt ccccatacag agattgcagg 11040
   ctgagaatga ctatatcctt gttaacagga ggtgggagca gggcacggta gctcacacct 11100
   gtaatcttgg cactttagga ggcggaggcg ggccgatcac ctgaagtaag gagttcgaga 11160
   ccagcctggc caacatgcaa agccctgtct ctactaaaaa tacaaaaatt agctgggtgt 11220
   ggtggtactc gcctgtaatc ccagctactc gggagactga ggcaggagaa tggcttgaac 11280
   ccggaaggta gaggttgcag tgagctgaga tcatgccact gtgctccagc ctaggtgaca 11340
   gagagagact ccatctcaaa aaaaaaaaaa aatacaggaa gggagttggg aatagggtgc 11400
   acatttagga agtcttgggg atttagtggt gggaaggttg gaagtccctc tctgattgtc 11460
   ttttcctcaa agaagtgcat ggctggtgtg gggtggggca ggagtgcttg ggttgtggtg 11520
   aaacattgga agagagaatg tgaagcagcc attcttttcc tgctccacag gaagccgagc 11580
   tgtctcagac actggcatgg tgttggggga gggggttcct tctctgcagg cccaggtgac 11640
   ccagggttgg aagtgtctca tgctggatcc ccacttttcc tcttgcagca gccagactgc 11700
   cttccgggtc actgccatgg aggagccgca gtcagatcct agcgtcgagc cccctctgag 11760
   tcaggaaaca ttttcagacc tatggaaact gtgagtggat ccattggaag ggcaggccac 11820
   caccccgacc ccaaccccag ccccctagca gagacctgtg ggaagcgaaa attcatggga 11880
   ctgactttct gctcttgtct ttcagacttc ctgaaaacaa cgttctggta aggacaaggg 11940
   ttgggctggg gacctggagg gctggggggc tggggggctg aggacctggt cctctgactg 12000
   ctcttttcac ccatctacag tcccccttgc cgtcccaagc aatggatgat ttgatgctgt 12060
   ccccggacga tattgaacaa tggttcactg aagacccagg tccagatgaa gctcccagaa 12120
   tgccagaggc tgctccccgc gtggcccctg caccagcagc tcctacaccg gcggcccctg 12180
   caccagcccc ctcctggccc ctgtcatctt ctgtcccttc ccagaaaacc taccagggca 12240
   gctacggttt ccgtctgggc ttcttgcatt ctgggacagc caagtctgtg acttgcacgg 12300
   tcagttgccc tgaggggctg gcttccatga gacttcaatg cctggccgta tccccctgca 12360
   tttcttttgt ttggaacttt gggattcctc ttcaccctta ggcttcctgt cagtgttttt 12420
   ttatagttta cccacttaat gtgtgatctc tgactcctgt cccaaagttg aatattcccc 12480
   ccttgaattt gggcttttat ccatcccatc acaccctcag catctctcct ggggatgcag 12540
   aacttttctt tttcttcatc cacgtgtatt ccttggcttt tgaaaataag ctcctgacca 12600
   ggcttggtgg ctcacacctg caatcccagc actctcaaag aggccaaggc aggcagatca 12660
   cctgagcccc aggagttcaa gaccagcctg ggtaacatga tgaaacctcg tctctacaaa 12720
   aaaatacaaa aaattagcca ggcatggtgg tgcacaccta tagtcccagc cactcaggag 12780
   gctgaggtgg gaagatcact tgaggccagg agatggaggc tgcagtgagc tgtgatcaca 12840
   ccactgtgct ccagcctgag tgacagagca agaccctatc tcaaaaaaaa aaaaaaagaa 12900
   aagctcctga ggtgtagacg ccaactctct ctagctcgct agtgggttgc aggaggtgct 12960
   tacacatgtt tgtttctttg ctgccgtgtt ccagttgctt tatctgttca cttgtgccct 13020
   gactttcaac tctgtctcct tcctcttcct acagtactcc cctgccctca acaagatgtt 13080
   ttgccaactg gccaagacct gccctgtgca gctgtgggtt gattccacac ccccgcccgg 13140
   cacccgcgtc cgcgccatgg ccatctacaa gcagtcacag cacatgacgg aggttgtgag 13200
   gcgctgcccc caccatgagc gctgctcaga tagcgatggt gagcagctgg ggctggagag 13260
   acgacagggc tggttgccca gggtccccag gcctctgatt cctcactgat tgctcttagg 13320
   tctggcccct cctcagcatc ttatccgagt ggaaggaaat ttgcgtgtgg agtatttgga 13380
   tgacagaaac acttttcgac atagtgtggt ggtgccctat gagccgcctg aggtctggtt 13440
   tgcaactggg gtctctggga ggaggggtta agggtggttg tcagtggccc tccgggtgag 13500
   cagtaggggg gctttctcct gctgcttatt tgacctccct ataaccccat gagatgtgca 13560
   aagtaaatgg gtttaactat tgcacagttg aaaaaactga agcttacgag gctaagggcc 13620
   tcccctgctt ggctgggcgc agtggctcat gcctgtaatc ccagcacttt gggaggccaa 13680
   ggcaggcgga tcacgaggtt gggagatcga gaccatcctg gctaacggtg aaaccccgtc 13740
   tctactgaaa aatacaaaaa aaaattagcc gggcgtggtg ctgggcacct gtagtcccag 13800
   ctactcggga ggctgaggaa ggagaatggc gtgaacctgg gcggtggagc ttgcagtgag 13860
   ctgagatcac gccactgcac tccagcctgg gcgacagagc gagattccat ctcaaaaaaa 13920
   aaaaaaaaag gcctcccctg cttgccacag gtctccccaa ggcgcactgg cctcatcttg 13980
   ggcctgtgtt atctcctagg ttggctctga ctgtaccacc atccactaca actacatgtg 14040
   taacagttcc tgcatgggcg gcatgaaccg gaggcccatc ctcaccatca tcacactgga 14100
   agactccagg tcaggagcca cttgccaccc tgcacactgg cctgctgtgc cccagcctct 14160
   gcttgccgct gacccctggg cccacctctt accgatttct tccatactac tacccatcca     14220
   cctctcatca catttccggc gggaatctcc ttactgctcc cactcagttt ccttttctct     14280
   ggctttggga cctcttaacc tgtggcttct cctcccacct cctggagctg gagcttaggc     14340
   tccagaaagg acaagggtgg ttgggagtag atggagcctg gttttttaaa tgggacaggt     14400
   aggacctgat ttccttactg cctcttgctt ctcttttcct atcctgagta gtggtaatct     14460
   actgggacgg aacagctttg aggtgcgtgt ttgtgcctgt cctgggagag accggcgcac     14520
   agaggaagag aatctccgca agaaagggga gcctcaccac gagctgcccc cagggagcac     14580
   taagcgaggt aagcaagcag gacaagaagc ggtggaggag accaagggtg cagttatgcc     14640
   tcagattcac ttttatcacc tttccttgcc tctttcctag cactgcccaa caacaccagc     14700
   tcctctcccc agccaaagaa gaaaccactg gatggagaat atttcaccct tcaggtacta     14760
   agtcttggga cctcttatca agtggaaagt ttccagtcta acactcaaaa tgccgttttc     14820
   ttcttgactg ttttacctgc aattggggca tttgccatca gggggcagtg atgcctcaaa     14880
   gacaatggct cctggttgta gctaactaac ttcagaacac caacttatac cataatatat     14940
   attttaaagg accagaccag ctttcaaaaa gaaaatagtt aaagagagca tgaaaatggt     15000
   tctatgactt tgcctgatac agatgctact tgacttacga tggagttact tctgataact     15060
   cgtcgtaagt tgaaatattg aaatattgta agttgaaaat ggatttaata cacctaatct     15120
   aaggaacatc atagcttagc ctagcctgct tttttttttt tttttttttt ggagacagag     15180
   tctcactctg ctacccaggc tggagtgcag tggcgggatc tcggctcact gcaacctccg     15240
   ccttctgggt tcaagcgatt ctcctgcctc agcccactga gtagctggga ttacaggcac     15300
   ctgccccgac gcccagctaa ttttttgtta tttatttcct ttttttttag tagagataga     15360
   atttcaccat gttggccagg ctagtctcga actcctgacc ttgtgatctg cctgccttgg     15420
   cctcccaaag tgctgggatt acaggcgtga gccaccgcac ctggcctgcc tagcctactt     15480
   ttattttatt tttaatggag acagcatctt gctctgttgc ccaggctgga ttacagtgat     15540
   gtgatcatag ctcattatac cctcctgggc tcaagcaatc cccctaactc tgcctcccca     15600
   gtagctagga ccacaggcat acaccaccat acccagctaa tttttaaaat tttttgtaga     15660
   tagatagagt ctcactatgt tgcccaggct ggtctctagc ctactttttt gagacaaggt     15720
   cttgctctgt cacccaggct ggatagagtg cagtagtgca gtcacagctc actgcagcct     15780
   ccacctccca ggctccatcc atcctcccag ctcagcctcc caagttgctt caactacagg     15840
   cctgcaccac catgcctggc taatttttat ttatttattt ttattttatt ttattttatt     15900
   ttttgagact cagtctcact ctgtcgcctt aggctggagt gcagtggcat gatctcggct     15960
   cactgctaac ctctgcctcc tgggtttcaa gtgattctcc tgcctcagcc tcccgaatag     16020
   ctaggactac aagcgcctgc taccacgccc ggctaatttg tgtattttta gtagagacag     16080
   ggtttcacca tgttggccag gctggtctcg aacttctgac catgtgatcg ccgcctcggc     16140
   ctcccaaagt gctgggatta caggtgtgag ccaccacgcc cggctaattt ttatttattt     16200
   atttaaagac agagtctcac tctgtcactc aggctagagt gcagtggcac catctcagct     16260
   cactgcagcc ttgacctccc tgggctccgg tgatttcacc ctcccaagta gctaggacta     16320
   caggcacatg ccacgacacc cagctaattt tttattttct gtgaagtcaa ggtcttgcta     16380
   cgttgcccat gctggtatca aacccctggg ctcaatcaat ccttccacct cagcctcccc     16440
   aagtattggg gttacaggca tgagctacca cactcagccc tagcctactt gaaacgtgtt     16500
   cagagcattt aagttaccct acagttgggc aaagtcatct aacacaaagc cctttttata      16560
   gtaataaaat gttgtatatc tcatgtgatt tattagatat tgttactaaa agtgagaaac     16620
   agcatggttg catgaaagga ggcacagtcg aagccaggca cagcctgggc gcagagcgag     16680
   actcaaaaaa agaaaaggcc aggcgcactc tcacgcctgt aatcccagca tttcgggagg     16740
   ctgaggcggg tggatcacct gaggtcagga gttcaagacc agcctagcca acatggtgaa     16800
   accccgtctc tactaaaata caaaaattaa ccgggcgtga tggcaggtgc ctgtaatccc     16860
   agctacttgg gaggctgagg caggagaatc gcttgaacca ggaggcggag gttgcaggga     16920
   gccaagacgg cgccactgca ctccagcctg ggcgatagag tgagactccg tctcagaaaa     16980
   aaaagaaaag aaacgaggca cagtcgcatg cacatgtagt cccagttact tgagaggcta     17040
   aggcaggagg atctcttgag cccaagagtt tgagtccagc ctgaacaaca tagcaagaca     17100
   tcatctctaa aatttaaaaa agggccgggc acagtggctc acacctgtaa tcccagcact     17160
   ttgggaggtg gaggtgggta gatcacctga cgtcaggagt tggaaaccag cctggctaac     17220
   atggtgaagc cccatctcta ctaaaaacac aaaaattagc cagtgtgaga cacgttgagt     17280
   ccacgtactc ggaggctgag gcacaagaat cacttgaacc ccagaggcgg agattcgaat     17340
   cagccaagat tgcaccattg cactcccgcc tgggcgacga gagtgagacc ccatctcaaa     17400
   ataaataaat aaatattttt aaaagtcagc tgtataggta cttgaagtgc agtttctact     17460
   aaatcgatgt tgcttttgat ccgtcataaa gtcaaacaat tgtaacttga accatctttt     17520
   aactcaggta ctgtgtatat acttacttct ccccctcctc tgttgctgca gatccgtggg     17580
   cgtgagcgct tcgagatgtt ccgagagctg aatgaggcct tggaactcaa ggatgcccag     17640
   gctgggaagg agccaggggg gagcagggct cactccaggt gagtgacctc agccccttcc     17700
   tggccctact cccctgcctt cctaggttgg aaagccatag gattccattc tcatcctgcc     17760
   ttcatggtca aaggcagctg accccatctc attgggtccc agccctgcac agacattttt     17820
   ttagtcttcc tccggttgaa tcctataacc acattcttgc ctccacgtag tatccacaga     17880
   acatccaaac ccagggacga gtgtggatac ttctttgcca ttctccgcca actccccagc     17940
   ccagagctgg agggtctcaa ggggcctaat aattgtgtaa tactgaatac agccagagtt     18000
   tcaggtcata tactcagccc tgccatgcac cggcaggtcc taggtgaccc ccgtcaaact     18060
   cagtttcctt atatataaaa tggggtaagg gggccgggcg cagtggctca cgaatcccac     18120
   actctgggag gccaaggcga gtggatcacc tgaggtcggg agtttgagcc cagcctgacc     18180
   aacatggaga aaccccatct ctactaaaaa tacaaaagta gccgggcgtg gtgatgcatg     18240
   cctgtaatcc cagctaccta ctcgggaggc tgaggcagga gaatcgcttg aacccgggag     18300
   gcagaggttg cggtgagctg agatctcacc attacactcc agcctgggca acaagagtga     18360
   aactccgtct caaaaaagta taataaagta aaatggggta agggaagatt acgagactaa 18420
   tacacactaa tactctgagg tgctcagtaa acatatttgc atggggtgtg gccaccatct 18480
   tgatttgaat tcccgttgtc ccagccttag gcccttcaaa gcattggtca gggaaaaggg 18540
   gcacagaccc tctcactcat gtgatgtcat ctctcctccc tgcttctgtc tcctacagcc 18600
   acctgaagtc caaaaagggt cagtctacct cccgccataa aaaactcatg ttcaagacag 18660
   aagggcctga ctcagactga cattctccac ttcttgttcc ccactgacag cctcccaccc 18720
   ccatctctcc ctcccctgcc attttgggtt ttgggtcttt gaacccttgc ttgcaatagg 18780
   tgtgcgtcag aagcacccag gacttccatt tgctttgtcc cggggctcca ctgaacaagt 18840
   tggcctgcac tggtgttttg ttgtggggag gaggatgggg agtaggacat accagcttag 18900
   attttaaggt ttttactgtg agggatgttt gggagatgta agaaatgttc ttgcagttaa 18960
   gggttagttt acaatcagcc acattctagg taggtagggg cccacttcac cgtactaacc 19020
   agggaagctg tccctcatgt tgaattttct ctaacttcaa ggcccatatc tgtgaaatgc 19080
   tggcatttgc acctacctca cagagtgcat tgtgagggtt aatgaaataa tgtacatctg 19140
   gccttgaaac caccttttat tacatggggt ctaaaacttg acccccttga gggtgcctgt 19200
   tccctctccc tctccctgtt ggctggtggg ttggtagttt ctacagttgg gcagctggtt 19260
   aggtagaggg agttgtcaag tcttgctggc ccagccaaac cctgtctgac aacctcttgg 19320
   tcgaccttag tacctaaaag gaaatctcac cccatcccac accctggagg atttcatctc 19380
   ttgtatatga tgatctggat ccaccaagac ttgttttatg ctcagggtca atttcttttt 19440
   tctttttttt tttttttttt ctttttcttt gagactgggt ctcgctttgt tgcccaggct 19500
   ggagtggagt ggcgtgatct tggcttactg cagcctttgc ctccccggct cgagcagtcc 19560
   tgcctcagcc tccggagtag ctgggaccac aggttcatgc caccatggcc agccaacttt 19620
   tgcatgtttt gtagagatgg ggtctcacag tgttgcccag gctggtctca aactcctggg 19680
   ctcaggcgat ccacctgtct cagcctccca gagtgctggg attacaattg tgagccacca 19740
   cgtggagctg gaagggtcaa catcttttac attctgcaag cacatctgca ttttcacccc 19800
   acccttcccc tccttctccc tttttatatc ccatttttat atcgatctct tattttacaa 19860
   taaaactttg ctgccacctg tgtgtctgag gggtgaacgc cagtgcaggc tactggggtc 19920
   agcaggtgca ggggtgagtg aggaggtgct gggaagcagc cacctgagtc tgcaatgagt 19980
   gtggactggg gggcccagtg cccgggttcc gggaggggaa caaaggctgg agactgggtc 20040
   agtctgcggg ctgcatgaca acaagggagg gggtggctcc attcataact caggaaccaa 20100
   ccgtccctcc tcccctccgc ccacggctgg cacaaggttc tctgcctccc ctgcttctag 20160
   gattgggctg cttccccctc ggcagcctct caccaaggat tacgggattt aaatgtcgtg 20220
   atttacgaag gctgagcctc cagggtggcc atcttcgtcc atcagaagtg gcaggatacc 20280
   tgggttccaa gggaacaggg tgg 20303
<210> 2
   <211> 36
   <212> DNA
   <213> artificial
<220>
   <223> primer
<220>
   <221> misc_feature
   <222> (1)..(36)
<400> 2
   gatcgatcga tcgatcttct ctgcaggccc aggtga 36
<210> 3
   <211> 20
   <212> DNA
   <213> artificial
<220>
   <223> primer
<220>
   <221> misc_feature
   <222> (1)..(20)
<400> 3
   ttctctgcag gcccaggtga 20
<210> 4
   <211> 21
   <212> DNA
   <213> artificial
<220>
   <223> primer
<220>
   <221> misc_feature
   <222> (1) .. (21)
<400> 4
   tcgcttccca caggtctctg c 21
<210> 5
   <211> 20
   <212> DNA
   <213> artificial
<220>
   <223> primer
<220>
   <221> misc_feature
   <222> (1)..(20)
<400> 5
   aaccccagcc ccctagcaga 20
<210> 6
   <211> 20
   <212> DNA
   <213> artificial
<220>
   <223> primer
<220>
   <221> misc_feature
   <222> (1)..(20)
<400> 6
   ccggggacag catcaaatca 20
<210> 7
   <211> 19
   <212> DNA
   <213> artificial
<220>
   <223> primer
<220>
   <221> misc_feature
   <222> (1)..(19)
<400> 7
   agggttgggc tggggacct 19
<210> 8
   <211> 20
   <212> DNA
   <213> artificial
<220>
   <223> primer
<220>
   <221> misc_feature
   <222> (1)..(20)
<400> 8
   gggatacggc caggcattga 20
<210> 9
   <211> 27
   <212> DNA
   <213> artificial
<220>
   <223> primer
<220>
   <221> misc_feature
   <222> (1)..(27)
<400> 9
   ccagttgctt tatctgttca cttgtgc 27
<210> 10
   <211> 18
   <212> DNA
   <213> artificial
<220>
   <223> primer
<220>
   <221> misc_feature
   <222> (1)..(18)
<400> 10
   ctggggaccc tgggcaac 18
<210> 11
   <211> 20
   <212> DNA
   <213> artificial
<220>
   <223> primer
<220>
   <221> misc_feature
   <222> (1)..(20)
<400> 11
   agctggggct ggagagacga 20
<210> 12
   <211> 19
   <212> DNA
   <213> artificial
<220>
   <223> primer
<220>
   <221> misc_feature
   <222> (1)..(19)
<400> 12
   ccggagggcc actgacaac 19
<210> 13
   <211> 20
   <212> DNA
   <213> artificial
<220>
   <223> primer
<220>
   <221> misc_feature
   <222> (1)..(20)
<400> 13
   aaaaggcctc ccctgcttgc 20
<210> 14
   <211> 19
   <212> DNA
   <213> artificial
<220>
   <223> primer
<220>
   <221> misc_feature
   <222> (1)..(19)
<400> 14
   aagcagaggc tggggcaca 19
<210> 15
   <211> 24
   <212> DNA
   <213> artificial
<220>
   <223> primer
<220>
   <221> misc_feature
   <222> (1)..(24)
<400> 15
   tgggacaggt aggacctgat ttcc 24
<210> 16
   <211> 23
   <212> DNA
   <213> artificial
<220>
   <223> primer
<220>
   <221> misc_feature
   <222> (1)..(23)
<400> 16
   ggcataactg cacccttggt ctc 23
<210> 17
   <211> 20
   <212> DNA
   <213> artificial
<220>
   <223> primer
<220>
   <221> misc_feature
   <222> (1)..(20)
<400> 17
   agcggtggag gagaccaagg 20
<210> 18
   <211> 22
   <212> DNA
   <213> artificial
<220>
   <223> primer
<220>
   <221> misc_feature
   <222> (1)..(22)
<400> 18
   tgccccaatt gcaggtaaaa ca 22
<210> 19
   <211> 23
   <212> DNA
   <213> artificial
<220>
   <223> primer
<220>
   <221> misc_feature
   <222> (1)..(23)
<400> 19
   tcgatgttgc ttttgatccg tca 23
<210> 20
   <211> 25
   <212> DNA
   <213> artificial
<220>
   <223> primer
<220>
   <221> misc_feature
   <222> (1)..(25)
<400> 20
   aatggaatcc tatggctttc caacc 25
<210> 21
   <211> 20
   <212> DNA
   <213> artificial
<220>
   <223> primer
<220>
   <221> misc_feature
   <222> (1)..(20)
<400> 21
   ggtcagggaa aaggggcaca 20
<210> 22
   <211> 20
   <212> DNA
   <213> artificial
<220>
   <223> primer
<220>
   <221> misc_feature
   <222> (1)..(20)
<400> 22
   tggcagggga gggagagatg 20
<210> 23
   <211> 19
   <212> DNA
   <213> artificial
<220>
   <223> primer
<220>
   <221> misc_feature
   <222> (1)..(19)
<400> 23
   ctctgactgc tcttttcac 19
<210> 24
   <211> 20
   <212> DNA
   <213> artificial
<220>
   <223> primer
<220>
   <221> misc_feature
   <222> (1)..(20)
<400> 24
   cattgaagtc tcatggaagc 20